# EUROPEAN PATENT SPECIFICATION

(11) **EP 1 284 296 B1**
(45) Date of publication and mention of the grant of the patent: **11.10.2006**
(21) Application number: 01929654.0
(22) Date of filing: 27.04.2001
(51) Int. Cl.: C12Q 1/68

(54) **METHOD FOR THE DETECTION OF MINORITY GENOMES IN VIRUS QUASISPECIES USING DNA MICROCHIPS**
METHODE ZUM NACHWEIS VON MINDERHEITSGENOMEN IN VIRALEN QUASI-SPEZIES UNTER VERWENDUNG VON DNA-MIKROCHIPS
METHODES PERMETTANT DE DETECTER DES GENOMES MINORITAIRES DANS DES QUASI-ESPECES VIRALES AU MOYEN DE MICROPUCES A ADN

(30) Priority: 27.04.2000 ES 200001068
(43) Date of publication of application: 19.02.2003
(73) Proprietor: CONSEJO SUPERIOR DE INVESTIGACIONES CIENTIFICAS, 28006 Madrid (ES)
(72) Inventor: ARIAS ESTEBAN, Armando, 28049 Madrid (ES); BARANOWSKI, Eric, 28049 Madrid (ES); BRIONES LLORENTE, Carlos, Centro de Astrobiologia, 28850 Torrejon de Ardoz (ES); DOMINGO SOLANS, Estebán, 28049 Madrid (ES); ESCARMIS HOMS, Cristina, 28049 Madrid (ES); GOMEZ CASTILLA, Jordi, Hospital Vall d'Hebron, 08085 Barcelona (ES); MARTIN RUIZ-JARABO, Carmen, 28049 Madrid (ES); PARRO GARCIA, Victor, Centro de Astrobiologia, 28850 Torrejon de Ardoz (ES)
(74) Representative: ABG Patentes, S.L.
(86) International application number: PCT/ES2001/000165
(87) International publication number: WO 2001/083815

(56) References cited:
- WO-A-01/07652
- US-A- 5 861 242
- WILSON J.W. ET AL.: 'Comparative evaluation of the human immunodeficiency virus genotyping systems: the HIV-genotypR method, the HIV PRT genechip assay and the HIV-1 RT line probe assay' J. CLIN. MICROBIOL. vol. 38, no. 8, August 2000, pages 3022 - 3028, XP002957070
- KOZAL ET AL.: 'Extensive polymorphisms observed in HIV-1 clade B protease gene using high-desity oligonucleotide arrays' NATURE MEDICINE vol. 2, no. 7, July 1996, pages 753 - 759, XP002928568
- TUAN VO-DINH ET AL.: 'Development of a DNA biochip: principle and applications' SENSORS AND ACTUATORS B 51 1998, pages 52 - 59, XP000669813
- HACIA ET AL.: 'Resequencing and mutational analysis using oligonucleotide microarrays' NATURE GENETICS SUPPLEMENT vol. 21, January 1999, pages 42 - 47, XP000865986
- NAJERA I. ET AL: 'Pol gene quasispecies of human immunodeficiency virus: Mutations associated with drug resistance in virus from patients undergoing no drug therapy' J. VIROLOGY vol. 69, 1995, pages 23 - 31

## Description

### FIELD OF THE TECHNIQUE

The invention refers to a method for the detection of minority genomes in viral quasispecies. The technique should be used for the genetic diagnosis of viral diseases by analysis of quasispecies of pathogenic viruses by using DNA microchips and other techniques.

### STATE OF THE ART

RNA viruses (that contain ribonucleic acid or RNA as the genetic material) have been associated with many diseases that affect humans, animals and plants and that cause high mortality and have considerable economic repercussions. The association of RNA viruses with important diseases is well known (see the chapters and references of the following books: Mohanty et al., 1981; Robart, 1995; Fields et al., 1996; Granoff et al., 1999; Flint et al., 2000). RNA viruses are known to be responsible both for acute (with symptoms of a few hours or days duration) and chronic or persistent infections (with or without apparent symptoms but with detectable virus for weeks, months, years or decades). Examples of acute human infections due to RNA viruses are: flu, measles, common cold, poliomyelitis, different kinds of viral encephalitis and haemorrhagic fevers and those associated with the Hepatitis A virus (a virus from the Pycnoviridae family). Examples of acute animal infections due to the RNA virus are foot-and-mouth disease, vesicular stomatitis, viral enteritis, Teschen disease, aviar encephalomyelitis and others. Examples of chronic human diseases due to RNA virus are Hepatitis C and AIDS (associated with human deficiency virus). Within this group can be included syndromes associated with the persistence of certain viruses after an acute infection. Examples of these are post-poliomyelitis syndrome (that results from the persistence of certain genomic variant forms of the poliomyelitis virus) and subacute sclerosant panencephalitis (that results from the persistence of variant genomic forms of the measles virus). Examples of chronic animal infections due to RNA viruses are meningitis caused by the infectious karyolymphomeningitis virus or equine anemia caused by the equine infectious anemia virus.

The genetic material of RNA viruses is RNA (this is the material that replicates inside infected cells and is responsible for the biological properties and the virulence of a virus). This contains information or a genetic message in the form of a polynucleotide produced by the polymerisation of four nucleotides (adenosine monophosphate (AMP), Cytosine monophosphate (CMP), Guanosine monophosphate (GMP) and Uridine monophosphate (UMP)). Polymers of these four monomers of a length ranging from 3,000 to 4,000 units constitute the genetic material of all RNA viruses described to date. During the infectious process (multiplication of a virus in cells or organisms), viral populations often form groups with a high number of infectious particles that can reach from 10⁵ to 10¹² infectious particles at the peak of an acute infection or at several moments during a chronic infection (Domingo et al., 1999d). The genetic message (represented in the nucleotide sequence of genomic viral RNA) is not identical in the individual genomes that make up the viral population and many individual genomes differ from the others in one or more positions. These comprise a group of very closely related but not identical sequences and this group of sequences is called a viral quasispecies. The concept of quasispecies applied to RNA viruses has a theoretical and an experimental basis.

The theoretical concept of quasispecies originated in a work by M. Eigen concerning a system of replicating molecules with continuous production of errors due to restricted accuracy in the recognition of template nucleotides (Eigen, 1971). In this first work, the concept of autoinstruction was distinguished from the general concept of autocatalysis. The concept of autoinstruction was proposed as necessary for a molecule to act as a template for replication. According to this, and in the case of RNA, nucleotides of the RNA template dictate the incorporation of its respective complements during the replication process. Thus, Adenine (A) is complementary to Uracyl (U) and Guanine (G) is complementary to Cytosine (C). A quality factor was defined that represents the fraction of the copying process that produces an exact copy of the template. When the accuracy of the copy is not equal to 1 (the maximum possible value) the copy of the main sequence, also called the master sequence, will produce some mistaken molecules that will have a certain probability of distribution. Therefore, depending on the accuracy of the copy there will be a different abundance of genomes with one, two, three or more differences with respect to the master sequence.

In this first work, M. Eigen refers to the comet's tail of erroneous copies whereas the terms quasispecies and mutant spectrum appeared later in the literature in works by M. Eigen and P.Schuster (Eigen et al., 1977; Eigen et al., 1978a; Eigen et al., 1978b; Eigen et al., 1979). In these works, the concept of selection equilibrium of the multiple variants generated in a copying process with limited accuracy is developed. This equilibrium is usually metastable, in the sense that it can collapse when a beneficial mutant appears in the population. This collapse produces a reorganization of the population variants and a new selection equilibrium point. As explained later, changes and fluctuations in the equilibrium of the genomic variants that constitute the population frequently occur in an RNA virus and are relevant to the invention presented here. Theoretical development of the concept of quasispecies has been described in several works and later reviews (Eigen et al., 1988; Eigen, 1992; Eigen 1996; Schuster et al.,1999; Domingo et al., 2000). The theoretical model of quasispecies represents the combination of the principles of Darwinian evolution and the information theory and is essential to understand the population dynamics of RNA viruses as described below.

The first experimental evidence that RNA viruses showed characteristics typical of quasispecies was obtained in works using the bacteriophage (bacterian virus) Qβ that infects certain strains of the bacteria *Escherichia coli* (Domingo et al., 1976; Batschelet et al., 1976; Domingo et al., 1978). The most significant observations were revealed by studying the reversion rate (conversion to the initial genomic sequence, also called wild type) of a mutant of the bacteriophage Qβ during its multiplication in *Escherichia coli*. This reversion rate was of the order of 10⁻⁴ substitutions per copied nucleotide, i.e. for each 10,000 times that the enzyme responsible for copying the mutant RNA of Qβ passed the mutated position of the template the product had a base different to the complementary base it was supposed to correspond. The experiments and calculations behind these deductions have been described by Domingo et al., and Batschelet et al., (Domingo et al.,1976; Batschelet et al., 1976). The second relevant observation was the discovery of high genetic heterogeneity (the presence of different mutant distributions in one or more positions) in the populations of bacteriophage Qβ (Domingo et al. 1978). The decisive experiments consisted of isolating the Qβ bacteriophage from an individual and isolated plaque that forms on the surface of a layer of susceptible *Escherichia coli* (plaque refers to the region of dead cells produced by the virus that multiplies starting from an individual initial infectious particle).

Classical virology experiments using dilutions of infected material have shown that with the bacteriophage Qβ and many other RNA viruses, a plaque develops from a single infectious RNA genome and not from several (this evidence has been summarised in Luria et al., 1978). When a virus from a plaque propagates in the bacteria *Escherichia coli* populations are formed that, when analysed by biological cloning techniques (isolation of individual well-separated viral plaques) proved to be genetically heterogeneous. The procedure consisted in labelling RNA viruses obtained from individual plaques with ³²P-Phosphate (neutral phosphate of which some of the normal phosphate ions had been substituted by the radioactive form or isotope-32) and RNA analysis after hydrolysis with Ribonuclease T1 (an enzyme that ruptures single chain RNAs in the positions occupied by guanylic acid GMP). The analysis consists in bidimensional electrophoresis (resulting from carrying out two successive electrophoreses with perpendicular electric fields) in which the positions of the radioactive stains that correspond to oligonucleotides T1 (the digestion products of viral RNA with ribonuclease T1) depend on their exact nucleotide composition. Therefore, the position of the oligonucleotides after bidimensional electrophoresis permits mutations to be detected and identified (De Watcher et al., 1972). RNA analysis of individual clones that had been derived from a single sequence of RNA revealed that most of these differed with respect to the original (parent) genome in one or more positions and only 14% of genomes were identical to the original one (Domingo et al. 1978). According to these results, the bacteriophage Qβ replicated with a high production of erroneous copies and its populations, instead of being homogeneous, contained dynamic distributions of variants.
As described in the original article (Domingo et al., 1978) : "A population of Qβ phage is in dynamic equilibrium with viable mutants, which, on one side appear with high frequency, and on the other side are negatively selected. The genome cannot be described as a unique sequence but rather as a weighted mean of a large number of individual sequences".

In later works using numerous RNA viruses of both animals and plants, all the viruses analysed have been shown to have the same characteristics of population structure as those described above for the bacteriophage Qβ. In the last two decades, methods for cloning viral genomes (biological and molecular) have improved and also procedures for rapid nucleotide sequenciation, including automated sequenciation (for explanatory manuals on the new DNA recombinant techniques *in vitro* and sequenciation see Sambrook et al., 1989; Howe et al., 1989; Heitman, 1993).

Application of these techniques to the molecular analysis of RNA genomes has resulted in determination of the quasispecies structure of RNA viruses that infect humans, animals and plants. Two types of results have confirmed the quasispecies nature of RNA viruses and of other genetic elements in which RNA is involved in the replication cycle: the calculation of high mutation rates by both genetic and biochemical techniques (rates usually range from 10⁻³ to 10⁻⁵ substitutions per copied nucleotide) and the direct demonstration of the presence of a mutant spectrum in the viral populations.

In addition to the RNA viruses, it has also been shown that viruses with a DNA genome for which in the infective cycle viral RNA is generated as one of the replicative intermediates, have also been shown to have a high mutation rate and quasispecies structure. Some of the viruses with a DNA genome and a quasispecies structure that infect humans and animals belong to the *Hepadnaviridae* family. The most well-known and studied of these is the human hepatitis B virus (HBV)(for general characteristics of hepadnaviruses consult Fields et al., 1966). It has been estimated that between 5 and 10% of the world population is a carrier of HBV and in some geographical regions such as Africa or Southeast Asia it is considered to be endemic (Maynard, 1990; Coleman et al.,1998). Between 5 and 10% of adults exposed to HBV become chronic carriers and can develop cirrhosis and cancer of the liver that is fatal in approximately half of these (Liaw et al., 1988).

There is considerable experimental evidence for the high mutation rates and heterogeneity in RNA populations, see Borrow et al., 1997; Borrow et al., 1998; Brions et al., 2000; Chen et al., 1996; Cornelissen et al., 1997; Domingo et al., 1993; Domingo, 1996; Domingo, 1997b; Domingo et al., 1999a; Domingo, 1999d; Domingo et al., 2000; Eigen, 1996; Escarmis et al.,1999; Escarmis et al., 1996; Flint et al., 2000; Granoff et al., 1999; Mateu et al., 1989; Morse, 1993; Morse, 1994; Mortara et al., 1998; Najera et al., 1995; Quiñones-Mateu et al., 1996a; Quiñones-Mateu et al., 1996b; Ruiz-Jarabo et al., 1999; Taboga et al., 1997; Weidt et al., 1995; and Weiner et al., 1995.

From these works it can be concluded that populations of viruses with RNA genomes and those that use RNA as an intermediate molecule in their replicative cycle have quasispecies behaviour and that this conduct is important for the adaptability, survival and pathogenicity of the virus. In the latest edition of the Virology Encyclopedia, 1999, the following generalised definition is given for quasispecies that is currently used in Virology: "Quasispecies are dynamic distributions of mutant recombinant genomes that are not identical but are closely related and that undergo a continuous process of genetic variation, competition and selection and operate as a unit of selection (Domingo, 1999a).
The quasispecies structure of RNA viruses (and those that use RNA as a replicative intermediate) has numerous biological implications that have been reviewed in many books and special editions (see for example Morse, 1993; Morse, 1994; Gibbs et al., 1995; Domingo et al., 1999d; Domingo et al., 2000). Some of the biological implications are relevant to this invention and are described below:
1) Viral quasispecies are reserves of genetic variants (and phenotypic; i.e. a variant of biological behaviour) that have a certain probability of being selected in response to a selection applied from outside the organism or endogenous selection from the infected organism itself (Domingo, 1996; Forns et al., 1999).
2) Among the variants that make up the mutant spectrum of a viral quasispecies there are mutants with a reduced sensitivity to inhibitors used in the treatment of viral diseases (Cornelissen et al., 1997; Najera et al., 1995; Lech et al., 1996; Quinones-Mateu et al., 1998; Havlir et al., 1996). The presence of minority variants with mutations that confer different degrees of resistance to inhibitors is one of the factors that contribute to therapeutic failure in the treatment of infections by human immunodeficiency virus (among the studies that demonstrate this finding are Richman, 1994; Domingo et al., 1997b; Palmer et al., 1999). For the human immunodeficiency virus there are catalogues of mutations that, either isolated or together, contribute to the low efficacy of antiretroviral treatments (Schinazi et al., 1997; Schinazi et al., 1999; Menendez-Arias et al., 1999).
3) The variants that comprise the mutant spectrum of a viral quasispecies include mutants with reduced sensitivity to antibodies or to cytotoxic T cells (CTLs) (see Borrow et al., 1997; Borrow et al., 1998; McMichael et al., 1997; Weidt et al., 1995; Weiner et al., 1995; Domingo et al., 1993; Taboga et al., 1997; Mortara et al., 1998).
4) In some cases direct proof has been obtained that the presence of antigenic variants or other types of variants with altered biological properties influence the progression of a viral disease *in vivo* (Pawlotsky et al., 1998; Forns et al., 1999; Evans et al. 1999).
As a consequence of the quasispecies structure of some viruses and their rapid diversification in nature, most pathogenic viruses circulate as different genomes that have been divided into types, subtypes, genotypes or biotypes and can require specific reagents for diagnosis. These subdivisions of a virus occur in important pathogenic viruses such as the human immunodeficiency virus, hepatitis C virus, the flu virus, human and animal rotaviruses, poliomyelitis virus, foot-and-mouth virus and many others (see, for example, Murphy, 1996, and the European patent application EP 0 984 067 A2).

Current techniques for the molecular diagnosis of viruses are based on the detection of majority viral genomes present in the population by using direct nucleotide sequencing, indirect methods of sequence detection (hybridisation of nucleic acids, polymorphisms revealed by using restriction enzymes on DNA copies of viral RNA or by changes in electrophoretic migration of heteroduplex produced by hybridisation of a reference DNA with the DNA copy of the genome being analysed etc.).
Sequenciation of biological or molecular clones can give an adequate genetic description of the quasispecies although only a restricted sample of genomes can be analysed for each viral quasispecies, usually no more than 20-30 clones (Briones et al., 2000). On the other hand, none of the techniques mentioned are very sensitive at detecting minority genomes within the quasispecies. For example, a minority genome can be located by analysis of consensus sequences provided that it is present in more than or equal to 30-50%. Certain hybridisation techniques of nucleic acids can detect minority genomes when these are present in more than 10-20% and by sequenciation of 20 molecular clones derived from the quasispecies (a slow and laborious technique) genomes present in more than or equal to 5% can be detected.

The recent development of DNA microarray technology, also called DNA microchips or chips (Southern et al., 1994; for a review see Nature Genetics 21, supplement, 1999) in which thousands of molecular probes, mainly oligonucleotides, can covalently bond to a solid support (glass, nitrocellulose, nylon etc.) has permitted the identification of polymorphisms of only one nucleotide in only one round of hybridisation. Moreover, this technique is much more sensitive than those mentioned in the previous paragraph and enables the detection of minority genomes that comprise only 1% of the total (Gerry et al., 1999).

DNA microchip technology exploits the technique developed by E. Southern (Southern, 1975) according to which nucleic acids can bind to a solid support and form stable hybrids with radioactive or fluorescent labelled complements. The stability of the hybrids depends on the degree of complementarity of the nucleotide sequences and on external factors such as the ionic strength of the medium, the pH or the temperature. It is possible to design and synthesize oligonucleotides, for example from 10 to 30 nucleotides, for which some of their positions, usually the central position, is different to that present in the complementary chain of a given gene. When the sequences of this gene are labelled with a radioactive or fluorescent compound and this is placed in contact with a set of nucleotides identical by four in four except for the central position that can be occupied by A,C,G or T and in specific conditions of ionic strength, pH and temperature, stable hybrids will only form where the pairing between complementary base pairs is complete. A positive result in the hybridisation immediately identifies the nucleotide present in the position of the gene under study (Hacia et al., 1998).

For the construction of a DNA microchip, one of two basic strategies can be followed: one of these consists in directly placing a previously synthesized probe on a solid support. The probe can be an oligonucleotide, a fragment amplified by PCR, a plasmid or a fragment of purified DNA. Another strategy consists in synthesizing the probes *in situ*, either by a method of photochemical deprotection of inactivated nucleotides by a photolabile substance (North American patent no. 6.022.963), by the ink jet method (Blanchard et al., 1996) or by physical confinement of the reagents (Maskos and Southern, 1993). To directly deposit the sample an automated system called an arrayer is used that can print up to 2,500 samples (100 micrometers diameter) per cm². By *in situ* synthesis by photochemical deprotection, 65,0000 points are easily achieved ("of 50 micrometers diameter) per cm².

DNA microchips can be used in gene expression studies, mainly resequencing genomes and genotyping. RNA expression can be analysed for thousands of genes using samples of diseased tissues (cancer, viral infections, bacterial infections etc.) or using the infectious agents themselves (virus, bacteria, fungi etc.). Discovery of the genes involved in these processes can help in the design and development of new drugs, diagnostic techniques etc. Resequenciation and genotyping studies can be used to discover mutations and nucleotide polymorphisms (SNP).

Several strategies have been described for the detection of SNPs. One of these is the one specified above (Hacia et al. 1998); The sequenciation strategy by hybridisation to a microchip of octa and decanucleotides amplified by the bonding of adjacent pentanucleotides (Parinov et al., 1996); new strategies that adapt enzymatic treatments such as that developed by Gerry et al. (1999), that combines polymerase chain reaction (PCR) and the ligase detection reaction (LDR) with a chip of universal code and that permits detection of mutations in human genomes that are present in less than 1% of the copy of the wild type DNA. Another technique uses the DNA polymerase activity of the Klenow fragment of the DNA polymerase of the *Escherichia coli* to elongate a hybridised oligonucleotide to another, that, in turn, acts as a template to extend the first one (Hacia, 1999). The design of the oligonucleotide can be such that for each four pairs of oligonucleotides the interrogant position corresponds to the first of the template that is to be copied (A,C,G or T) and if the extension reaction is carried out in the presence of the four dideoxynucleotides (ddNTPs) labelled with a different fluorescent compound then it is possible to discern, by the type of fluorescence, which is the nucleotide in the interrogant position. Following this methodology, it is possible to synthesize *in situ* double chain oligonucleotides that can be used to study protein-DNA interactions (Bulyk et al., 1999) and, therefore, open up a new range of possibilities for the discovery of new diagnostic techniques and drugs.

Another area in which the microchips can be applied is that of identification of microorganism species, mainly variants or strains (more or less virulent) of the same species (Gingeras et al., 1998), either for traditional applications (resistance to drugs, toxins, pathogenicity factors etc.) or for ecological applications (biodiversity, polymorphic dispersion etc.). Gingeras et al. made a DNA microchip with oligonucleotides interrogating all the positions (of the two chains) of a DNA fragment of 705 bp of the rpoB gene of Mycobacterium tuberculosis, to study, in a collection of 63 clinical isolates of M. tuberculosis, the existence of mutations that confer resistance to Rifampicin. The identification of species was based on the existence of specific polymorphisms of species that can be easily determined with a DNA microchip.

Another example of the use of DNA microchips to identify bacteria was described in the North American patent no. 5.925.522, in which Wong et al. describe techniques for the detection of Salmonella using DNA chips with specific oligonucleotide sequences.

Development of the technology of DNA chips started only recently (1996) but is progressing at a startling rate. This enables better and more accurate detection of genetic alterations in complex mixtures that was previously only possible using laborious and lengthy techniques. With a well-designed DNA microchip, point mutations can be identified in a few hours whereas this takes days or even weeks by conventional methods.

### DESCRIPTION

### BRIEF DESCRIPTION

The invention relates to a method for designing an individual antiviral therapy for a subject based on the detection of minority memory genomes in a viral quasispecies responsible for viral resistance to a drug or drug combination.

The solution provided by this invention is based on the discovery of the existence of viral memory genomes, that, in spite of being minority genomes, reflect the evolutionary background of the virus in the infected organism and includes the analysis of viral quasispecies, by any appropriate technique, eg. using DNA microchips, Heteroduplex Trace Assay (HTA) and molecular cloning.

The method described in this invention permits, among other applications, to detect and identify minority memory genomes; to study viral quasispecies responsible for viral resistance to drugs, or concerned with selection against defence systems (immune response) of the infected organism and to design individualized therapeutic regimes.

Example 1 describes the detection and characterisation of minority genomes of the foot-and-mouth disease virus (FMDV) using DNA microchips; Example 2 describes detection and characterisation of minority genomes of the human immunodeficiency virus (HIV) that are carriers of mutations with resistance to Zydovudine (also called azidothymidine, AZT) by DNA microchips. Example 3 describes the detection and characterisation of memory genomes in populations of FMDV; Example 4 describes the detection of memory genomes in HIV subjects who are carriers of mutations that confer resistance to drugs in treated patients. Example 5 describes the detection of memory genomes in quasispecies of the hepatitis C virus (HCV); and Example 6 describes the detection of memory genomes of the hepatitis B virus (HBV) in carriers of mutations that confer resistance to drugs in treated patients.

### DETAILED DESCRIPTION OF THE INVENTION

### Definitions

The term "quasispecies" refers to a viral population comprised of dynamic distributions of mutant and recombinant genomes that are not identical but are closely related, that undergo a continuous process of genetic variation, competition and selection and that operate as a selection unit.

The term "memory genome" of a quasispecies refers to the capacity of the quasispecies to maintain above the base threshold specific mutant genomes, which, some time throughout the evolutionary history of the quasispecies in an infected organism, corresponded to the majority sequence or the mean of the quasispecies.

The "base threshold" can be defined as the proportion with which the different genomes of the majority and memory genomes appear in the quasispecies. This proportion is approximately 10⁻⁵ (0.001%) in the quasispecies.

The "majority or mean genome" is defined as the viral genome with the nucleotide sequence that represents 50% or more of the quasispecies.

"Genome type" or "virus type" is the genome of a viral isolate with a sequence that is considered as wild-type by the scientific community and serves as a reference genome for that species. In the case of HIV-1, the strain HXB2 (accession number in the genbank data base: ko3455) is considered as the wild type genome.

"Minority genome" is the viral genome with a nucleotide sequence that is present in less than 50% of the quasispecies. A genome can be minority in one quasispecies and majority in another and vice versa.

Memory genome refers to the nucleotide sequence of a viral genome that presents at least one mutation in comparison to the majority gnome of a specific quasispecies and provides information about the evolutionary history of that quasispecies.

As used in this description "minority memory genome" refers to the nucleotide sequence of a viral genome that presents at least one mutation in relation to the majority genome of a specific quasispecies, provides information about the evolutionary history of that quasispecies and is present in less than 50%.

The term "nucleic acid" refers to a deoxyribonucleic sequence, peptide-nucleic or ribonucleic acid, with a length greater than or equal to 4 nucleotides, abbreviated nt, that can be single or double stranded.
An "oligonucleotide" is defined as a single band DNA molecule of between 4 and 250 nt long that can hybridise with a complementary DNA sequence.

The term "nucleotide position" refers to the site that each of the nucleotides occupies in a nucleic acid sequence.
"Encoding sequence" is the nucleotide sequence that specifies the amino acid sequence of a protein.

A "codon" or "triplet" is a sequence of three consecutive nucleotides that specify an amino acid within an encoding sequence. For example, the triplet ATG specifies or encodes the amino acid methionine (M).
A mutation is an alteration of the nucleotide sequence of a nucleic acid with respect to another reference sequence. This alteration can correspond to the substitution of one nucleotide by another, an insertion or a deletion of one or more nucleotides.

In the present description "memory oligonucleotide (MO) of a quasispecies" is defined as a nucleic acid from 4 to 250 nucleotides long that is equal or complementary to a majority or mean viral genome sequence except for at least one nucleotide. A memory nucleotide can also be a majority nucleotide of another quasispecies.
"Memory Oligonucleotide type 1 (MO1)" can be defined as a nucleic acid from 4 to 250 nucleotides in length that is equal and/or complementary to a majority or mean viral genome except for 1 to 6 central positions.
"Memory oligonucleotide type 2 (MO2)" refers to nucleic acids from 5-50 nt long that are formed by stacking two oligonucleotides after hybridising with another complementary nucleic acid of the virus. One of the stacked oligonucleotides is formed by a combination of four oligonucleotides that differ (i.e. have an interrogant position) in the position immediately adjacent to the anterior oligonucleotide and have a different fluorescent marker covalently bound to the other end.

"Stacking of bases" or "stacking of nucleotides" refers to the interaction established between two adjacent bases or the ends of two oligonucleotides by stacking their purines or pyrimidines. In this type of interaction, covalent bonds are not formed between adjacent bases but this interaction increases the stability of the bonding of both nucleotides to the chain that is complementary to both of them. In this way, for example, an oligonucleotide of 5 nt can remain stably bound to its complementary chain only if stacking takes place with an adjacent oligonucleotide of 5 nt or more.
"Interrogated position" is the nucleotide position of the viral genome sequence for which the composition is unknown.
"Interrogant or discriminatory position" is the nucleotide position of the memory oligonucleotide occupied by one of the four possible nucleotides (A,C,G,T).

"Memory oligonucleotide type 3 (MO3)" is a nucleic acid from 5 to 250 nt with two parts, the 5' section of the oligonucleotide is complementary to another oligonucleotide that is absent from the viral genome and the 3' section is complementary to the viral genome, the last position is an interrogant position.
"Memory oligonucleotide type 4 (MO4)" is a nucleic acid from 5 to 250 nt with a length complementary to the viral genome that has a fluorescent substance covalently bound to the 3'end. An MO3 nucleotide can be used together with an MO4 nucleotide to detect mutations by the PCR/LDR technique described by Gerry et al., (1999), using a DNA microchip with oligonucleotides complementary to the 5'part of MO3.
"Memory oligonucleotide type 5 (MO5)" is a nucleic acid of 5-250 nt with a length complementary to the viral genome in which the last position on the 3'end is anterior to an interrogated position of the viral genome. "Memory oligonucleotide type 6 (MO6)" is a nucleic acid of 5 to 250 nucleotides complementary to a sequence with a majority genome of a viral quasispecies with insertions of 1 to 10 nucleotides in relation to this sequence of the majority genome.
"Memory oligonucleotide type 7 (MO7)" is a nucleic acid of 5 to 250 nucleotides complementary to sequence of a majority genome of the viral quasispecies with deletions of 1-10 nucleotides with respect to this sequence of the majority genome.
"Memory oligonucleotide type 8 (MO8)" is a nucleic acid of 5 to 250 nucleotides complementary to a mutant sequence previously described in the database.
The term "probe" refers to nucleic acids of 5 to 250 base pairs in length comprised by specific nucleotide sequences that permit total or partial hybridisation with complementary target sequences under certain conditions.
"Target sequences" are sequences of nucleic acids susceptible to hybridisation with the oligonucleotide probes. In the present invention target sequences are labelled with a radioactive or fluorescent substance when DNA microchip techniques are used but not when this is done by heteroduplex trace assay (HTA)(Gerotto et al., 1999).
"Flanking sequences" in an oligonucleotide are those (5 to 100 nt) that accompany the interrogated position/s that, together, permit hybridisation of the oligonucleotide to wild type or memory (mutant) genomes.
"Wild type oligonucleotide" is that which has total sequence identity with the wild-type or corresponding genome.
"Mutant oligonucleotide" is that with total sequence identity with the corresponding wild type genome except for in the interrogant positions in which they have the sequence that corresponds to a mutant strain in this position.
The term "hybridisation" refers to a process via which, in certain conditions, two complementary chains of nucleic acids join in an antiparallel way, by forming hydrogen bridges to form double chain nucleic acids according to the rules of pairing between nitrogenated bases.
"Total hybridisation" or "100% hybridisation" refers to the hybridisation that takes place when all the nucleotides of a probe or oligonucleotide pair with a target sequence or vice versa.
A "mismatch" occurs when in at least one site of a double chain nucleic acid, both chains have two non-complementary nucleotides.
A "hybrid" is the result of the hybridisation process between two single chain nucleic acids. The dispairings in the central positions have a greater destabilizing effect than when these are located on the ends. Stability depends on the length, the number of dispairings and external factors such as temperature, ionic strength of the medium and pH. The greater the length, the fewer dispairments, lower ionic strength, lower temperature and a pH close to neutral all increase the stability of the hybrid.
"Nested PCR" is a method for the enzymatic amplification of DNA that consists in carrying out two successive
rounds of PCR, the second with a pair of oligonucleotides interior to those used in the first PCR. By carrying out two rounds of PCR it is possible to amplify extremely small amounts of initial DNA which can be very useful in clinical samples in which the virus is often present in very low quantities.
"A sample suspected to contain the viral quasispecies" is any sample from an animal, plant, bacteria or cell culture that can be infected with at least one viral quasispecies.

### Method for the detection of minority genomes

The invention uses a technique for detecting minority genomes of a nucleic acid population of a viral quasispecies, present in less than 50% and containing at least one mutation in comparison to the majority genome of this quasispecies, hereinbelow, method of the invention comprising:
a) Extracting the nucleic acid of the viral quasispecies from a sample suspected to contain viral quasispecies.
b) Amplifying at least one nucleic acid fragment of this viral quasispecies and
c) Detecting and analysing the existence of minority genomes using techniques based on DNA microchips, heteroduplex trace assay (HTA) and molecular cloning.

### Alternative 1

In one particular embodiment the invention provides a method to detect minority genomes that includes the use of DNA microchips. More specifically, the invention describes a technique to detect minority genomes from a population of nucleic acids of a viral quasispecies present in less than 50% and containing at least one mutation in comparison to the majority genome of this quasispecies. It consists of the following stages:
a) Extraction of a nucleic acid of this viral quasispecies from a sample with suspected contents of this viral quasispecies;
b) Amplification of at least one nucleic acid fragment of this viral quasispecies;
c) Labelling the amplified fragment or fragments with a marker substance;
d) Construction of a DNA microchip such that points are produced that include:
   i) at least one oligonucleotide that can serve as a positive control;
   ii) at least one oligonucleotide that can serve as a negative control
   iii) at least one memory oligonucleotide and
   iv) means that can be used to plot a calibrated curve
e) To place in contact said fragments amplified in stage b) and labelled in stage c) with the oligonucleotides present in the DNA microchip prepared in stage d) under conditions that permit hybridisation only when all the nucleotides of an oligonucleotide present in this DNA microchip pair with a nucleotide sequence present in these amplified and labelled fragments;
f) To identify the oligonucleotides present in this DNA microchip that have hybridised with these amplified and labelled fragments, ruling out negative hybridisations or background noise and
g) To select the oligonucleotides present in this DNA microchip that have hybridised with these amplified and labelled fragments and that by interpolation with the calibration curve show a proportion of these fragments in the DNA quasispecies lower than 50% characteristic of minority genomes.
In one particular embodiment, these minority genomes are minority memory genomes that can be present in the viral quasispecies in a proportion of between 0.1 and 10% of the quasispecies.

The viral quasispecies can correspond to a virus with a DNA genome or a virus with an RNA genome. In one application this viral quasispecies belongs to a virus selected from the group formed by the human immunodeficiency virus type 1 (HIV-1), the human immunodeficiency virus type 2 (HIV-2), the hepatitis C virus (HCV) and the hepatitis B virus (HBV).

The technique of the invention starts with extraction of the nucleic acid of the viral quasispecies from the sample suspected to contain the viral quasispecies, for example a clinical sample or a sample selected from a viral culture. Extraction of the nucleic acid is done by conventional techniques (Sambrook et al., 1989).

Amplification of the fragment or fragments of nucleic acid extracted from the viral quasispecies can be done by any conventional method. In one particular embodiment, this amplification is done by enzymatic techniques, for example, by polymerase chain reaction (PCR), ligase chain reaction (LCR) or amplification based on transcription (TAS). If the nucleic acid extracted is RNA, reverse transcription is carried out (RT) by conventional techniques of the viral RNA previous to the amplification stage b). In a practical embodiment of the technique described here, amplification of the fragment or fragments of the nucleic acids extracted from the viral quasispecies is done by RT-PCR (when starting with an RNA virus not integrated in the host cell DNA) or by simple PCR or nested PCR (for virus DNA or in the case of virus RNA integrated in the genome of the host cells). The same fragment is amplified from an isolate of the wild type virus strain (wild strain).

Although the fragment or fragments of nucleic acid to be amplified that are extracted from a viral quasispecies can proceed from any gene or region of the viral genome, this fragment or fragments will preferentially proceed from all or at least part of a gene that is essential for replication or persistence of the virus in the infected organism. In one particular embodiment this fragment or fragments come from an essential gene selected from the group formed by the protease fragment (PR) of the *pol* gene of HIV, the reverse transcriptase fragment (RT) of the HIV gene pool, the integrase fragment of the HIV gene pool, the *env* gene of HIV, the *gag* gene of HIV, the gene of the non-structural protein NS5A of HCV, the region between nucleotides 175-215 of HVC, the region between nucleotides 310-350 of HCV, and the reverse transcriptase (RT) fragment of the *pol* gene of HBV.

The amplified fragments in general are purified and labelled with an appropriate marker compound such as a radioactive substance for example ³²P, ¹³P etc. a fluorescent compound for example Cy3, Cy5 etc. or a compound detectable by colorimetric reaction, for example a compound that produces a coloured enzymatic reaction. In general, two different DNA fragments are labelled; one with a sequence exactly identical to the wild type virus and another comprised of the combination of different sequence fragments from the quasispecies to be studied.

In one particular embodiment, the nucleic acid fragments amplified, for example by RT-PCR or PCR, from viral RNA or DNA, are labelled with nucleotide precursors that carry a fluorochrome, for example Cy3 or Cy5-dCTP or Cy3 or Cy-dUTP, either including this in the oligonucleotides used as primers in the RT-PCR amplification reaction, by random labelling using hexanucleotide extension or by chemical labelling with alternating reagents such as psoralene-biotin. In a preferential embodiment, as mentioned previously, it is appropriate to label two samples, one containing the wild type sequence labelled with fluorochrome as a control (reference sample) and another containing the sequences to be studied labelled with another fluorochrome (sample test). The possibility of labelling with different fluorochromes permits hybridisations to be done with the two samples at the same time in one microchip.

Then, a DNA microchip was constructed with points comprising:
i) at least one oligonucleotide that serves as a positive control
ii) at least one oligonucleotide that serves as a negative control
iii) at least one memory oligonucleotide and
iv) means that can allow to plot a calibrated curve.

The oligonucleotides used as controls permit the quality or the quantity of the hybridisation to be determined with the objective of being able to correlate the intensity of the hybridisation signal with the abundance of a mutation in a viral quasispecies.

As a positive control, an oligonucleotide can be used with a sequence that is 100% complementary to a known sequence region of the majority genome or the wild type genome.

The negative control can be selected from the group formed by: i) an oligonucleotide with a sequence complementary to a region of known sequence of the majority genome of the wild type virus except in at least one position; ii) an oligonucleotide with a sequence that is complementary to a region of known sequence of the majority genome or of the wild type genome except for one interrogant position; and iii) an oligonucleotide with a sequence that is complementary to a known sequence of the majority genome or the genome of the wild-type virus except for the interrogant position and at least one nucleoside that flanks the interrogant position. The memory oligonucleotide is selected from the group comprised of the memory oligonucleotides identified as MO1, MO2, MO3, MO4, MO5, MO6, M07,and MO8 and combinations of these.

The calibrated curve is made up of a series of nucleotide combinations in variable and known proportions, one of these is 100% complementary to a known sequence region of the majority genome or the wild type genome and the other is an oligonucleotide that differs from the previous one in at least one position. In one particular embodiment, this calibrated curve is made from a series of mixtures of oligonucleotides in variable and known proportions, one of these is 100% complementary to a known sequence region of the majority genome or the wild type genome and the other is an oligonucleotide that differs from the previous one in the interrogant position.

The DNA microchip can be constructed using conventional techniques. In one particular embodiment said DNA microchip is composed of previously synthesised oligonucleotide points, whereas in another particular embodiment, said DNA microchip is composed by oligonucelotide points synthesised *in situ*. In one particular embodiment DNA microchips were constructed that contained memory oligonucleotides that interrogate each of the viral genome sites to be studied. In other words, each interrogated position of the viral genome is represented by four different points in the microchip each of which contains an interrogant oligonucleotide for each base A,C,G or T. Other types of oligonucleotides are also included that serve as controls of the quality or quantity of hybridisation in order to be able to correlate the intensity of the hybridisation signal with the abundance of a mutation in a viral quasispecies. In another particular embodiment, the possibility of constructing DNA microchips with memory oligonucleotides is contemplated with more than one interrogant position (2 or 3) per codon analysed. In the same way, memory oligonucleotides can be included interrogating by insertions and deletions.

The DNA fragments amplified in stage b) and labelled in stage c) (target nucleic acids) are placed in contact with the oligonucleotides present in the DNA microchip prepared in stage d) under conditions that permit hybridisation, only when all the nucleotides of an oligonucleotide present in the DNA microchip pair with a nucleotide sequence present in the labelled and amplified fragments, in other words, in conditions in which only oligonucleotide sequences complementary with 100% of the sequences of the amplified and labelled fragments hybridise. Selection of appropriate hybridisation conditions depends on several factors that include size of the oligonucleotide and that these can be easily fixed in each case by a specialised laboratory technician.

Once the hybridisation is complete, it must be confirmed that this has taken place and the oligonucleotides present in the DNA microchip are identified, ruling out negative hybridisations or background noise. In one specific embodiment, if the microchip has been constructed with MO1, MO2 or oligonucleotides complementary to the 5'portion of MO3, after hybridisation this is washed with an appropriate buffer solution, in appropriate conditions, and the result of the hybridisation is confirmed by DNA microchip scanning with a scanner equipped with a confocal microscope and at least two lasers that emit light of different wavelengths and filters that correspond to the fluorochromes used to label the amplified DNA fragments, and computer equipment that can produce a computer image of the hybridisation result. Certain programmes permit the intensity of the hybridisation to be quantified and can draw up calibration curves using the standards of known concentration included in the DNA microchip.

Finally, the results are interpreted. The hybridisation pattern obtained indicates the presence or absence of minority genomes, for example minority memory genomes in the viral quasispecies for each of the interrogated positions. Therefore, in the analysis of clinical samples it is possible to determine:
i) whether, in the viral quasispecies, there are genomes with nucleotide mutations in the codons involved in resistance to antiviral drugs. The presence of these mutations in the majority genome would orientate towards the current pattern of resistance usually derived from the antiviral therapy that the patient is receiving at the time of study. The presence of these mutations in minority memory genomes would result from the patients' previous history of antiviral treatment. In any case, the presence of resistance mutations in genomes that are well or less well represented (majority or memory) in the viral quasispecies will produce a lack of response to the corresponding drug from that moment on. Therefore, this drug should be excluded (both as monotherapy and in the combination of two or more drugs) in future therapeutic protocols designed by the doctor or the veterinary surgeon to suppress or reduce viral replication; and
ii) the presence in viral quasispecies of genomes (majority or minority) with nucleotide mutations in the codons involved in immune system escape. In this way, the doctor or veterinary surgeon can take appropriate measures related to the use of specific antibodies, vaccines or other treatments the action of which is based on their effect on the immune system.
   The invention contemplates the possibility of quantifying minority genomes, in particular minority memory genomes. To do this:
   1) a microchip was designed that had:
      a) controls of memory and wild type oligonucleotides known in duplicate
      b) combinations of memory and wild type oligonucleotides: a) in different proportions, mutant/wild-type, for example 10⁻⁵ , 10⁻⁴, 10⁻³, 10⁻², 10⁻¹, 1, 10, 10², 10³, 10⁴, 10⁵ and
   2) hybridisation of the microchip is done by:
      a) the sample test and the reference test (fragment of the same length and equal sequence to the wild type) labelled with different fluorochromes. Quantification is done by differences in hybridisation intensity with the set of oligonucleotides mentioned previously in 1(b); and
      b) the sample test and a mixture made up of pure amplified fragments of the wild-type sequence and a known memory sequence in different proportions and labelled with the same fluorochrome that is different to that used in the sample test. To plot a calibration curve it is necessary to carry out the hybridisation process once for each concentration to be studied.

The washed microchips can be read using a scanner equipped with a confocal microscope and two lasers that emit light with different wavelengths. In this way it is possible to simultaneously read two fluorochromes in the same microchip.

Determination of the intensity of hybridisation in minority or memory oligonucleotides by comparison with controls immediately identifies the mutations that can form part of a minority or memory genome.

### Alternative 2

In another specific embodiment, the invention provides a method to detect minority genomes that includes use of techniques based on heteroduplex trace assay (HTA) (Gerotto et al., 1999). This alternative to the invention method can be used to monitor viral quasispecies in the same patient. More specifically, the invention provides a method to detect minority viral genomes in a population of nucleic acids of a viral quasispecies present in a proportion of less than 50% and containing at least one mutation in relation to the majority genome of this quasispecies that consists in:
a) extracting the nucleic acid from the viral quasispecies from a sample with suspected contents of this viral quasispecies.
b) amplification of at least one fragment of nucleic acid of this viral quasispecies;
c) cloning the DNA fragments amplified in b) in a suitable vector
d) determination of the majority genome sequence of a viral quasispecies for the amplified fragment.
e) amplification of the DNA fragments cloned in stage c) and labelling of the amplified fragments with a marker compound;
f) place in contact, in a hybridisation reaction, the amplified and labelled fragments from stage e) with the fragments directly amplified from the nucleic acid of the viral quasispecies from stage b); and
g) resolve the different viral sequences and identify the mutations indicative of the minority genomes present in the viral quasispecies.

Extraction of the nucleic acid and amplification of the fragment or fragments of nucleic acid of this viral quasispecies is done as mentioned previously in the alternative method of the invention that used DNA microchips.

Cloning of fragments of nucleic acid and sequenciation of fragments and sequences can be done by conventional techniques known by specialised laboratory technicians. In a specific embodiment, cloning of the DNA fragments amplified in stage b) was done in a plasmid with a large number of copies. Information about cloning techniques and sequenciation of nucleic acid sequences can be found in Sambrook et al., 1989.

For the marker compound used in stage e) to label DNA fragments amplified and cloned in stage c) any appropriate marker can be used. In a specific embodiment, the 5'ends of these DNA fragments are labelled with polynucleotide kinase and [γ-³²P] -ATP.

After the hybridisation reaction, the resolution of the different viral sequences can be done by:
g.i) Fractionation of the hybrids formed in stage f) by polyacrylamide gel electrophoresis in non-denaturising conditions;
g.ii) Identification of the existence of minority genomes by the number of mutations in relation to the sudden change in electrophoretic mobility;
g iii) extraction of DNA hybridised and fractionated by polyacrylamide gel electrophoresis by elution;
g iv) amplification of the fragments eluted in stage g iii);
g v) sequenciation of the fragments amplified in stage g iv); and
g vi) comparison of the sequences deduced in stage v) and identify mutations indicative of the minority genomes present in the viral quasispecies.

According to this alternative, the existence of nucleotide changes between the labelled probe and the target DNA are revealed by a delay in electrophoretic mobility of the heteroduplex with formed dispairments. As an homoduplex migration control the probe is hybridised with its own unlabelled sequences such that the difference in migration with the heteroduplex is proportional to the number of nucleotide changes. It is, therefore, possible to detect memory genomes and to calculate the number of nucleotide changes in the region studied in each one. A DNA microchip that interrogates all the sites of the DNA fragments studied will give the identity of each nucleotide.

### Alternative 3

In another particular embodiment, the invention provides a method to detect minority genomes that includes determination of the consensus sequence of the viral quasispecies or molecular cloning followed by sequenciation of other clones obtained. More specifically, the invention provides a method to detect minority genomes of a nucleic acid population of a viral quasispecies present in a proportion of less than 50% and containing at least one mutation in relation to the majority genome of this quasispecies comprising:
a) Extracting the nucleic acid of this viral quasispecies from a sample suspected to contain this viral quasispecies.
b) Amplifying at least one fragment of nucleic acid of this viral quasispecies
c) Determining the majority genome sequence of the viral quasispecies for this amplified fragment.
d) Optionally, cloning the nucleic acid fragment amplified in a vector;
e) Sequencing the cloned fragment and
f) Comparing the sequences deduced in stages c) and e) and identifying the mutations indicative of the minority genomes present in the viral quasispecies.
Extraction of the nucleic acid and amplification of the fragment or fragments of nucleic acid of this viral quasispecies is done similarly to the procedure described previously in relation to the alternative method of the invention that concerns the use of DNA microchips.

Cloning of nucleic acid fragments and the sequenciation of fragments and sequences can be done by conventional methods known by technicians skilled in this area. Information about techniques for cloning and sequencing sequences can be found in Sambrook et al.,1989.

The majority viral genome sequence and its later comparison by alignment of sequences, for example using PILEUP or CLUSTAL, will give accurate information about the existence of minority genomes and the precise mutations that these characterise.

One valuable aspect of the invention concerns the utilisation of information about the existence of viral minority memory genomes to design new individual antiviral therapies. As indicated previously, the presence of a memory genome that is a carrier of a mutation that, from data in the literature or previous studies, is known to be associated with the resistance of a drug would imply the recommendation to not use this drug in the therapy that the patient is prescribed from that moment on. The continued use of a drug or drugs for which a viral quasispecies has developed resistance mutations would hinder the action of the antiviral drug that would, nevertheless, continue to produce side effects, of variable degrees of severity, in the patient. Therefore, determination of the profile of mutations of resistance to antiviral drugs produces an improvement in the quality of life of the patient (by eliminating the side effects of one of the drugs to which the virus has developed resistance) and the important reduction in economic costs for the Heath Service that normally finances the medication (the drug or drugs that, in spite of their inactivity against the virus, could still be administered if the resistance of the virus to this/these drugs was unknown). In this context, one of the main practical applications of the invention lies in the considerable increase in the amount of information available about resistance mutations for each individual patient since, in addition to the mutations present in the majority genome (the only ones determined by current conventional techniques), information is also available about minority memory genomes in viral quasispecies that can also reduce (in the short or medium-term) efficacy of the drug.

Another important application of the technique of the invention is related with the study and follow-up of the phenomenon of memory of a quasispecies in an infected organism and its correlation with the population dynamics of a virus.

An interesting advantage of the technique of the invention is that it permits minority genomes to be quantified, especially memory minority genomes present in a viral quasispecies by incorporation of the appropriate controls, for example:
[1] Series in triplicate of mixtures at different known concentrations of sequence oligonucleotides equal to the wild type virus and/or to the majority sequence of the quasispecies and to a mutant sequence. A characteristic series contains an oligonucleotide of the mutant virus in proportions of 1, 5.10⁻¹, 10⁻¹, 5.10^{-2,} 10⁻², 5.10⁻³, 10⁻³, 5.10⁻⁴, 10⁻⁴, 5.10⁻⁵, 10⁻⁵ and 0 in relation to a wild type oligonucleotide. After hybridisation with a sample labelled with the wild type virus and then scanned a pattern of decreasing intensities is obtained such that all the points that remain in the linear region of the curve permit a calibration line to be drawn and from this a direct relationship can be established between the hybridisation intensity and the proportion of molecules in relation to the total number. The intensity of the hybridisation signal given by the minority oligonucleotides, for example, the minority memory oligonucleotides, with interrogant positions after hybridisation of the test sample labelled with another fluorochrome, can be correlated with the values obtained in the reference curve;
[2] Quadruplicate points comprised by equal oligonucleotides at the same concentrations, complementary to the oligonucleotides contained in different concentrations in a labelling mixture. Neither these oligonucleotides nor their complementary ones should form part of the fragment amplified by PCR of the wild type virus and the test sample. The correlation between the intensity of the hybridisation signal and that obtained for minority oligonucleotides, for example minority memory oligonucleotides, hybridised with the test sample, permit the proportion of the mutant genomes present in the quasispecies studied and
[3] Oligonucleotide series with interrogant positions with complementary sites, but not the flanking sequences, are absent from the wild type labelling mixtures. The mean of the hybridisation intensities given for these series makes up the "background noise" of the hybridisation.

An additional advantage of the invention is that it permits minority genomes arising from phenomena different to the memory genome but that can have important implications in the persistence of the virus in the organism to be identified.

The invention also provides a kit for the detection of minority genomes present in the viral quasispecies that includes at least one oligonucleotide that serves as a positive control, at least one that serves as a negative control, at least one memory oligonucleotide selecting from oligonucleotides identified as MO1, MO2, MO3, MO4, MO5, MO6, MO7 and MO8 and means that can be used to draw up a calibration curve. The different oligonucleotides present in the kit are usually found covalently bound and in an organised manner in a DNA microchip. The interrogant position of the memory oligonucleotides refers to the mutations responsible for the resistance to drugs or escape mutants of the defence system of the infected organism. Memory oligonucleotides are also incorporated with interrogant positions of apparently silent mutations that can confer resistance to drugs that the virus has not yet been exposed to or that could produce by combination with later mutations, new epitopes not recognised by the immune system. Likewise, the kit provided by the invention can include a set of oligonucleotides for the amplification by RT-PCR and/or by PCR or by nested PCR the fragments of the viral genome sequence where the mutations are to be located. The kit provided by the invention can also contain all or some of the reagents required to carry out the method described in the invention including appropriate buffer solutions or standards, instructions, protocols, practical advice with a problem shootout section and suitable packaging.

In one specific embodiment, the kit provided by the invention is a kit to detect minority genomes, preferentially minority memory genomes, present in quasispecies of HIV-1, HIV-2, HCV and HBC. As an example, the memory oligonucleotides that contain the interrogant positions of the mutations shown in Table II (see Example 2) permit memory mutations to be identified associated with resistance in genes that encode the protease (PR) and reverse transcriptase (RT) of HIV. The present invention includes the use of all the oligonucleotides with interrogant positions in each of the three positions of the codons of the PR genes and RT of HIV that appear in Table II. In another specific embodiment, the invention includes the use of all the oligonucleotides with interrogant positions for all the positions included in regions 1978-2010 and 6625-6744 (taking A of the ATG of the polyprotein as position 1) and 175-350 (in relation to position 1 of the viral genome) and the HCV type 1-b.

### Manufacture of DNA microchips

The manufacture of DNA microchips containing interrogant oligonucleotides for the identification of minority genomes, for example minority memory genomes, present in viral quasispecies is done by conventional techniques. In general, the interrogant oligonucleotides can be obtained from commercial laboratories by chemical synthesis. The solid support of the microchip can be a glass microscope slide onto which an automated device places sample of between 0.2 and 30 nl of oligonucleotide containing from 2 to 300 fmol and forming a spot of 50 to 250 µm diameter. The microchip should contain probes that identify the copy type (as the control), probes that identify the majority and minority genomes (or memory) and probes that serve as a negative control, i.e. that do not hybridise either with the master copy or with the minority genomes (or memory). Similarly, this also includes a calibration curve drawn up using known concentrations of labelled target sequences as described previously. The number of oligonucleotides present in the chip depends on the number of interrogated positions. In general, at least 10 interrogant oligonucleotides are required for each of the codons of the interrogated genes (see Table 1), one oligonucleotide for the master copy and three with the remaining interrogant positions for each of the positions of the codon. Therefore, a microchip to be used to seek minority or memory genomes with mutations in 200 possible positions should have at least 2,000 probes. Nevertheless, since it is expensive to synthesize large amounts of nucleotides the number of points on the microchip can be considerably reduced if memory genomes of a known sequence are required (such as those shown in Table II), or if a preliminary study is carried out to eliminate the possible mutations that do not produce amino acid changes in the synthesized protein. A typical design for MO1 is shown in Table I.

**Table I**

| Design of Memory Oligonucleotide Type 1 (MO1) | | |
|---|---|---|
| Mutation | Oligonucleotide | |
| a) For only one mutation: | | |
| ATG→TTG | NNNNNNNTACNNNNN | wt |
| | NNNNNNNNAACNNNN | mut |
| | | |
| b) For mutations in the three positions: ATG→NTG, ANG, ATN | | |
| NNNNNNNTACNNNNN | | |
| NNNNNNNAACNNNNN mut1 | | |
| NNNNNNNCACNNNNN mut2 | | |
| NNNNNNNGACNNNNN mut3 | | |
| | | |
| NNNNNNNTACNNNNN | | |
| NNNNNNNTCCNNNNN mut4 | | |
| NNNNNNNTGCNNNNN mut5 | | |
| NNNNNNNTTCNNNNN mut6 | | |
| | | |
| NNNNNNNTACNNNNN | | |
| NNNNNNNTAANNNNN mut 7 | | |
| NNNNNNNTAGNNNNN mut 8 | | |
| NNNNNNNTATNNNNN mut 9 | | |

where:
- N represents any of the four nucleotides (A,C,G,T)
- wt is the wild-type
- mut is the mutant
There are different protocols to fix DNA to the support and to prepare it for hybridisation with the labelled target samples. Hybridisation of the test samples and the reference samples with the microchip probes is carried out under specific conditions such that stable hybrids are formed with probes for which the complementarity is 100%. These hybridisation conditions depend on the type of minority or memory oligonucleotides present in the microchip. For example, for MO2, to join the first of the oligonucleotides a hybridisation buffer can be used that is comprised of NACl 1M, EDTA 1 mM, Tween 20 1% and sodium phosphate 5mM, pH 7.0 (Parinov et al., 1996) and the hybridisation time is 15 minutes at 0°C. The DNA that has not joined on is washed with the same hybridisation buffer at the same temperature for 10-20 seconds.
The union of the second oligonucleotide is carried out at 0°C for 5 minutes in the same hybridisation buffer and the unbound oligonucleotides are washed off with the same solution at 20°C for 10 minutes. The conditions of the second washing are more limiting because the hybrids formed by the stacking of the two oligonucleotides are more stable in these conditions.

The hybridisation and washing conditions should be optimised for each type of oligonucleotide. The kit of this invention includes the standards, protocols and everything necessary to provide the conditions required for the correct utilization of the kit. Similarly, the kit also includes items necessary to establish the correct conditions to carry out enzymatic modifications to detect memory mutations by PCR/LDR. The possibility of enzymatically modifying the microchip after the hybridisation in order to improve its signal/noise ratio has also been catered for.

The following is a list of the literature references cited in the text:
Allen, M., Deslauriers, M., Andrews, C., et al., (1998).
   Identification and characterization of mutations in hepatitis B virus resistant to lamivudine. Hepatology 27: 1670-1677.
Baranowski, E., Sevilla, N,. Verdaguer,N., Ruiz-Jarabo, C.M. Beck, E. and Domingo, E. (1998). Multiple virulents of foot-and-mouth disease virus in cell culture. J. Virol 72, 6362-6372.
Batschelet, E., Domingo, e. and Weismann, C. (1976). The proportion of revertant and mutant phage in a growing population as a function of mutation and growth rate. Gene 1, 27-32.
Blanchard, A.P., Kaiser, R.J. and Hood, L.E. (1996). Synthetic DNA arrays. Biosensors and Bioelectronics 11, 687-690.
Boom, R,. Sol, C., Salimans, M., et al., (1990). Rapid and simple method for purification of nucleic acids, J. Clin. Microbiol. 28: 495-503
Borrow P,. Lewicki, H., Wei, X., Horwitz, M.S., Peffer, N., Meyers, H., Nelson, J.A.Gairin, J.E.Hahn B.H., Oldstone, M.B. and Shaw, G.M. (1997). Antiviral pressure exerted by HIV-1 specific cytotoxic T lymphocytes (CTLs) during primary infection demonstrated by rapid selection of CTL escape virus. Nat. Med. 3, 205-211.
Borrow, P., and Shaw, G.M. (1998) Cytotoxic T-lymphocyte escape viral variants: how important are they in viral evasion of immune clearance in vivo? Immunol. Rev. 164, 37-51.
Briones, C., Mas, A., Gomez Mariano, G, Altisent, C., Menendez Arias, L., Soriano, V., and Domingo, E. (2000) Dynamics of dominance of a dipeptide insertion in reverse transcriptase of HIV-1 from patients subjected to prolonged therapy. Virus Res. 66, 13-26.
Bulyk, M. L. Gentalen, E. Lockhart, D.J. and Church, M. 1999. DNA-protein interactions by double-stranded DNA arrays. Nature Biotech. 17, 573-577.
Cabot, B., Martell, M., Esteban J.I. Sauleda S., Otero, T., Esteban, R., Guardia, J, and Gomez, J., 2000. Nucleotide and amino acid complexity of hepatitis C virus qausispecies in serum and liver. J. Virol. 74, 805-811.
Chen Z, Luckay A, Sodora DL, et al. (1997), Human immunodeficiency virus type 2 (HIV-2) seroprevalence and characterisation of a distinct HIV-2 genetic subtype from the natural range of simian immunodeficiency virus-infected sooty mangabeys, J. Virol. 7: 3953-3960.
Chen, W., and Baric, R. S. (1996). Molecular anatomy of mouse hepatitis virus persistence: coevolution of increased host cell resistance and virus virulence. J. Virol 70, 3947-3960.
Clavel F, Guetard, D, Brun-Vezinet F, et al. (1986), Isolation of a new human retrovirus from West African patients with AIDS, Science 233: 343-346.
Coleman, P., McQuillan, G., Moyer, L., et al. (1998). Incidence of hepatitis B virus infection in the United States, 1976-1994: estimates from the National Health and Nutrition Examination Surveys. J. Infect. Dis. 178: 954-959.
Cornelissen, M., Van den Burg, R., Zorgdrager, F., Lukashov, V., and Giudsmit, J. (1997). Pol gene diversity of five human immunodeficiency virus type 1 subtypes: evidence for naturally occurring mutations that contribute to drug resistance, limited recombination patterns, and common ancestry for subtypes B and D. Virol. 71, 6348-6358.
De Antoni A, Foli A, Lisziewicz J, et al., (1997), Mutations in the pol gene of human immunodeficiency virus type-1 in infected patients receiving didanosine and hydroxyurea combination therapy, J. Infect. Dis. 176: 899-903.
De Jong JJ, Goudsmit J, Lukasov VV, et al. (1999). Insertion of two amino acids combined with changes in reverse transcriptase containing tyrosine B215 of HIV-1 resistant to multiple nucleoside analogs, AIDS 13: 75-80.
De watcher, R., and Fiers, W. (1972). Preparative two-dimensional polyacrylamide gel electrophoresis of 32P-labelled RNA. Anal. Biochem. 49, 184-197.
Domingo, E., Flavell, R.A., and Weismann, C. (1976). In vitro site-directed mutagenesis: generation and properties of an infectious extracistronic mutant of bacteriophage Qβ. Gene 1, 3-25.
Domingo, E, Sabo, D., Taniguchi, T. and Weismann, C (1978). Nucleotide sequence heterogeneity of an RNA phage population. Cell 13, 735-744.
Domingo, E., Diez, J., Martinez, M.A., Hernandez, J., Holguin, A., Borrego, B. M., and Mateu, M. G. (1993). New observations on antigenic diversification of RNA viruses. Antigenic variation is not dependent on immune selection [published erratum appears in J. Gen Virol 1994 Apr; 75 (Pt 4): 949]. J. Gen Virol 74, 2039-2045.
Domingo, E. (1996). Biological significance of viral quasispecies. Viral Hepatitis Reviews 2, 247-261.
Domingo, E. (1997b). RNA virus evolution, population dynamics and nutritional status. Biol. Trace Elem. Res 56, 23-30.
Domingo E. (1999a) Quasispecies. In "Encyclopedia of Virology" (A. Granoff, and R. G. Webnster eds.) pp. 1431-1436. Academic Press, London.
Domingo E., Webster R.G. and Holland, J. J., Eds. (1999d). Origin and Evolution of Viruses. San Diego Academic Press.
Domigo, E., Biebricher, C., Holland J.J. and Eigen, M (2000). Quasispecies and RNA virus evolution: principles and consequences. Landes Bioscience, Austin.
Eigen, M. (1971). Self-organization of matter and the evolution of biological macromolecules. Naturwissenschaften 58, 465-523.
Eigen, M. (1992). Steps towards life. Oxford University Press.
Eigen, M. (1996). On the nature of virus quasispecies. Trends Microbiol. 4, 216-218.
Eigen, M. and Schuster, P. (1977). The hypercycle: a principle of natural self-organisation. Part A: Emergence of the hypercycle Naturwissenschaften 64, 541-565.
Eigen, M. and Schuster, P. (1978a). Part B: The hypercycle: a principle of natural self-organization. Part B: The abstract hypercycle. Naturwissenschaften 65, 7-41.
Eigen, M. and Schuster, P. (1978b). The hypercycle: a principle of natural self-organisation. Part C: The realistic hypercycle, Naturwissenschaften 341-369.
Eigen, M. and Schuster, P. (1979). The hypercycle. A pripciple of natural self-organization. Springer.
Eigen, M., McCaskill, J. and Schuster, P. (1988). Molecular quasispecies. J. Phys. Chem. 92, 6881-6891.
Escarmis, C., Davila, M., and Domingo, E., (1999). Multiple molecular pathways for fitness recovery of a virus debilitated by operation by Muller's ratchet. J. Mol. Biol. 285, 495-505.
Escarmis, C., Davila, M., Charpentier, N., Bracho, A., Moya, A., and Domingo, E. (1996). Genetic lesions associated with Muller's ratchet in an RNA virus. J. Mol. Biol. 264, 255-267.
Evans, D. T., O'Connor, D. H., Jing, P., Dzuris, J. L., Sidney J., da Silva, J., Allen, T.M., Horton, H., Venham, J.E., Rudersdorf, R.A., Vogel, T., Pauza, C.D., Bontrop, R. E., DeMars, R., Sette, A., Hughes, A.L. and Watkins, D.I. (1999). Virus-specific cytotoxic T-lymphocyte responses select for amino acid variation in simian immunodeficiency virus Env. And Nef. Nature Medicine 5, 1270-6.
Fields, B. N. Knipe, D. M., Howley, P. M., Chanock, R. M, Melnick, J. L., Monath, T. P., Roizman, B., and Straus, S. E., Eds. (1996). Field's virology 3^{rd} edition. Philadelphia: Lippincott-Raven.
Flint, S.J., Enquist, L. W., Krug, R.M., Racaniello, V. R., and Skalka, A. M. (2000). Virology. Molecular Biology. Pathogenesis and Control. ASM Press, Washington, D.C.
Forns, X., Purcell, R.H. and Bukh, J. (1999). Quasispecies in viral persistence and pathogenesis of hepatitis C virus. Trends Microbiol. 7, 402-410.
Gao F. et al. (1998), A comprehensive panel of near-full-length clones and reference sequences for non-subtype B isolates of human immunodeficiency virus type-1, J. Virol. 72: 5680-5698.
Gerotto M., Sullivan DG, Polak SJ, et al. (1999), Effect of retreatment with interferon alone or interferon plus rivabirin on Hepatitis C virus quasispecies diversification in non-responder patients with chronic hepatitis C, J. Virol. 73: 7241-7247.
Gerry N.P. Witowski, N. E., Day, J. Hammer, R.P., Barany, G. and Barany, F. (1999). Universal DNA microarray method for multiplex detection of low abundance point mutations. J. Mol. Biol. 292, 251-262.
Gibbs, A., Calisher, C., and Garcia-Arenal, F., Eds. (1995). Molecular Basis of Virus Evolution. Cambridge: Cambridge University Press.
Granoff A., and Webster R.G. Eds. (1999). Encyclopedia of Virology 2^{nd} edition. San Diego: Academic Press.
Hacia J.G., Sun., Hunt N., et al (1999), Strategies for mutational analysis of the large multi-exon ATM gene using high-density oligonucleotide arrays, Genome Research 8:1245-1258
Hacia, J. G. (1999). Resequencing and mutational analysis using oligonucleotide microarrays. Nature Genetics 21, 42-47.
Havlir, D. V., Eastman, S., Gamst, A., and Richman, D. D. (1996). Neviparine-resistant human immunodeficiency virus: kinetics of replication and estimated prevalence in uyntreated patients. J. Virol. 70, 7894-7899.
Heitman, J. (1993). On the Origins, structures and functions of restriction-modification enzymes. In "Genetic Engineering" (J.K.Setlow Ed.) Vol. 15, pp. 57-108. Plenum Press, New York.
Hertogs K, Bloor S, de Vroey V, et al., (2000), A novel human immunodeficiency virus type I reverse transcriptase mutational pattern confers phenotypic lamividine resistance in the absence of mutation 184V, Antimicrob. Agents Chemother. 44: 568-573.
Howe, C, J., and Ward, E.S. (1989). Nucleic Acids Sequencing: A Practical Approach. IRL Press, Oxford.
Innis, M.A., Gefland, D.H. Sninsky, J.J, and White, T.J. PCR Protocols. A guide to methods and applications. Academic Press, San Diego, 1990.
Janssens W, Heyndrickx L, Van der Auwera G et al., (1999), Interpatient genetic variability of HIV-1 group O, AIDS 13: 41-48.
Korber B, Kuiken C, Foley B, Hahn B, McCutchan F, Mellors JW and Sodroski J, Human retroviruses and AIDS 1988, Los Alamos National Laboratory, Los Alamos, New Mexico, USA, 1999; regular updates on http://hiv-web.lanl.gov.
Liaw, Y.F.Tai, D.I., Chu, C.M. et al, (1988). The Development of Cirrhosis in patients with chronic type B hepatitis: a prospective study. Hepatology 8: 493-496.
Luria, S.E., Darnell, J., F.E., Baltimore, D., and Campbell, A. (1978). General Virology, 2^{nd} ed. John Wiley and Sons, New York.
Martinez, M. A, Verdaguer, N., Mateu, M.G. and Domingo, E. (1997) Evolution subverting essentiality: dispensability of the cell attachment Arg-Gly-Asp motif in multiply passaged foot-and-mouth disease virus. Proc Natl Acad Sci USA 94, 6798-6802.
Mas A, Quiñones-Mateu ME, Domingo E and Soriano V (1999), Phylogeny of HIV type 1 group O isolates based on env gene sequences, AIDS Res. Hum. Retrov. 15: 769-773.
Maskos U and Southern E. M. 1993. A novel method for the analysis of multiple sequence variants by hybridisation to oligonucleotide arrays. Nucleic Acid Res. 21, 2267-2268.
Mateu, M.G., Martinez, M.A., Rocha, E., Andreu, D., Parejo, J., Giralt, E., Sobrino, F., and Domingo, E (1989). Implications of a quasispecies genome structure: effect of frequent, naturally occurring amino acid substitutions on the antigenicity of foot-and-mouth disease virus. Proc. Natl Acad Sci USA 86, 5883-5887.
Mateu, M. G., Roche, E., Vicente, O., Vayreda, F., Navalpotro, C., Andreu, D., Pedroso, E., Giralt, E., Enjuanes, L., and Domingo, E. (1987). Reactivity with monoclonal antibodies of viruses from an episode of foot-and-mouth disease. Virus Res. 8, 261-274.
Maynard, J.E. (1990). Hepatitis B: global importance and need for control. Vaccine 8: S18-S20.
McCutchan, F, Salminen M, Carr J and Burke, D (1996), HIV-1 genetic diversity, AIDS 10: S13-S20.
McMichael, A.J. and Philips, R.E. (1997),. Escape of human immunodeficiency virus from immune control. Annu Rev. Immunol. 15, 271-296.
Menendez-Arias, L., and Domingo E., (1998). Resistance tables for antiretroviral drugs, AIDS Cyber J. 1: 95-127.
Menendez-Arias, L., and Domingo, E., (1999). Cambios de aminoacido asociados a resistencia a inhibidores de la retrotrascriptasa y la protease el VIH. In "Guia práctica para el manejo clinico de las resistencias al VIH" (B. Clotet, L. Ruiz, X. Martinez-Picado, L. Menendez-Arias, J. M. Gatell, and D. D. Richman, Eds.), p. 215-267. Grupo E, Enthers, Merch Sharp and Dohme de España, S.A. Mohanty, S.B. and Dutta, S.K. (1981). Veterinary Virology. Lea & Febiger, Philadelphia.
Morse, S.S. Ed. (1993). Emerging Viruses: Oxford University Press, Oxford.
Morse, S.S., Ed. (1994). The Evolutionary Biology of Viruses. New York: Raven Press.
Mortara, L., Letourneur, F., Gras-Masse, H., Venet, A., Guillet, J.G., and Bourgault-Villada, I. (1998). Selection of virus variants and emergence of virus escape mutants after immunization with an epitope vaccine. J Virol 72 1403-1410.
Murphy, G. A. (1996). Virus taxonomy. In "Field's virology" (B.N. Fields, D.M. Knipe, P.M. Howley, R.M. Chanock, J.L. Melnick, T.P. Monath, B.Roizman and S.E.Straus, Eds.), p. 15-59. Lipincott-Raven Publishers, Philadelphia Pa.
Najera, I., Holguin, A., Quiñones-Mateu, M.E., Munoz-Fernandez, M.A.Najera, R. Lopez-Galindez, C, and Domingo, E. (1995). Pol gene quasispecies of human immunodeficiency virus: mutations associated with drug resistance in virus from patients undergoing no drug therapy. J. Virol. 69, 23-31.
Palmer, S. Shafer, R.W. and Merigan, T.C. (1999). Highly drug resistant HIV clinical isolates are cross-resistant to many antoretroviral compounds in current clinical development. AIDS 13, 661-7.
Paraskebis D and Hatzakis A (1999), Molecular epidemiology of HIV-1 infection, AIDS rev. 1: 238-249.
Parinov, S., Barsky, V., Yershov, G. Kirillov, E., Timofeev E, Belgovskiy, A. and Mirzabekov, A. 1996. DNA sequencing by hybridisation to microchip acto and decanucleotides extended by stacked pentanucleotides. Nucleic Acid Res. 24, 2998-3004.
Pawlotsky, J. M. Germanidis, G., Neumann, A. U., Pellerin, M. Frainais, P.O. and Dhumeaux, D. (1998). Interferon resistance of hepatitis c virus genotype 1b: relationship to non-structural 5A gene quasispecies mutations. J. Virol. 72, 2795-2805.
Quiñones-Mateu, M.E. Holguin A., Dopazo, J., Najera, I and Domingo, E. (1996a). Point mutation frequencies in the pol gene of human immunodeficiency type-1 are two- to threefold lower than those of env. AIDS Res Hum Retroviruses 12, 1117-1128.
Quiñones-Mateu, M. E., Holguin, A., Soriano, V., and Domingo, E (1996 b). env gene diversity of HIV type 1 isolates from Spain. AIDS Res Hum Retroviruses 12, 955-957.
Ratner L, Haseltine W, Patarca R, et a., (1985). Complete nucleotide sequence of the AIDS virus, HTLV-III, Nature 313: 277-284.
Richman, D. D. (1994). Resistance, drug failure and disease progression. AIDS Res Hum Retroviruses, 10, 901-5.
Robart, H. A. (1995). Human Enterovirus infections. ASM Press, Washington, D.C.
Robertson D, Sharp P, McCutchan and Hahn B (1995), Recombination in HIV-1, Nature 374: 124-126.
Ruiz-Jarabo, C.M., Sevilla, N., Davila, M., Gomez-Mariano, G., Baraowski, E., and Domingo, E (1999). Antigenic properties and population stability of a foot-and-mouth disease virus with an altered Arg-Gly-Asp receptor recognition motif.J. Gen. Virol. 80, 1899-909.
Sambrook, J., Fritsch, E.F. and Maniatis, T. (1989). Molecular cloning. A laboratory manual. Cold Spring Harbor Laboratory Press, New York.
Schinazi R.F., Larder B.A. and Mellors J.W.(1999), Mutations in retroviral genes associated with drug resistance: 1999-2000 update, Internatl. Antiviral News 7: 46-69.
Schinazi R.F., Larder B.A. and Mellors J.W.(1997). Mutations in retroviral genes associated with drug resistance. Int. Antivir. News 5, 129-142.
Schuster, P., and Stadler, P.F. (1999). Nature and Evolution of early replicons. In "Origin and Evolution of Viruses" (E. Domingo, R.G. Webster, and J.J. Holland Eds.) p.1-24. Academic Press, San Diego.
Simon F., Mauclere P., Roques P et a., (1998), Identification of a new human immunodeficiency virus type 1 distinct from group M and group O, Nat. Med. 4: 1032-1037.
Southern E.M. (1975) Detection of specific sequences among DNA fragments separated by gel electrophoresis. J. Mol. Biol. 98 (3): 503-17.
Southern et al., 1994. Arrays for complementary oligonucleotides for analysing the hybridisation behavior of nucleic acids. Nucleic Acid Res. 22, 1368-1373, 1994.
Stocker, M., and Macpherson, I., 1964. Nature. 203. 1355-1357.
Taboga, O., Tami, C., Carrillo, E., Nuñez, J. I., Rodriguez, A., Saiz, J.C., Blanco, E., Valero M.L., Roig, X., Camarero, J.A., Andreu, D., Mateu, M.G., Giralt, E., Domingo, E., Sobrino, F. and Palma, E.L. (1997). A large-scale evaluation of peptide vaccines against foot-and-mouth disease: lack of solid protection in cattle and isolation of escape mutants. J. Virol. 71: 2606-2614.
Takwhisa J., Zekeng L., Ido E. et al., (1994). Human immunodeficiency virus type-1 intergroup (M/O) recombination in Camerron, J. Virol. 73: 6810-6820.
Widt, G., Deppert, W., Utermohlen, O., Heukeshoven, J., and Lehmann-Grube, F. (1995). Emergence of virus escape mutants after immunization with epitope vaccine. J. Virol. 69, 7147-7151.
Weiner, A., Erickson, A.L. Kansopon, J., Crawford, K., Muchmore, E., Hughes, A.L. Houghton, M., and Walker, C.M. (1995). Persistent hepatitis C virus infection in a chipanzee is associated with emergence of a cytotoxic c lymphocyte escape variant. Proc. Natl. Acad. Sci.USA 92, 2755-2759.
Winter M.A., Cooley KL, Girard Y.A. et al., (1998). A 6-basepair insert in the reverse transcriptase gene of human immunodeficiency virus type 1 confers resistance to multiple nucleoside inhibitors, J. Clin. Invest. 192:1769-1775.

### DESCRIPTION OF THE FIGURES OF THE INVENTION

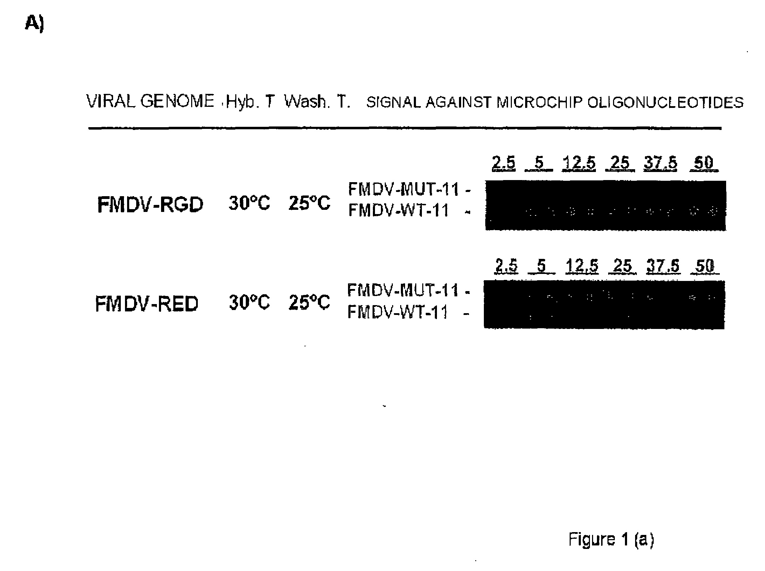

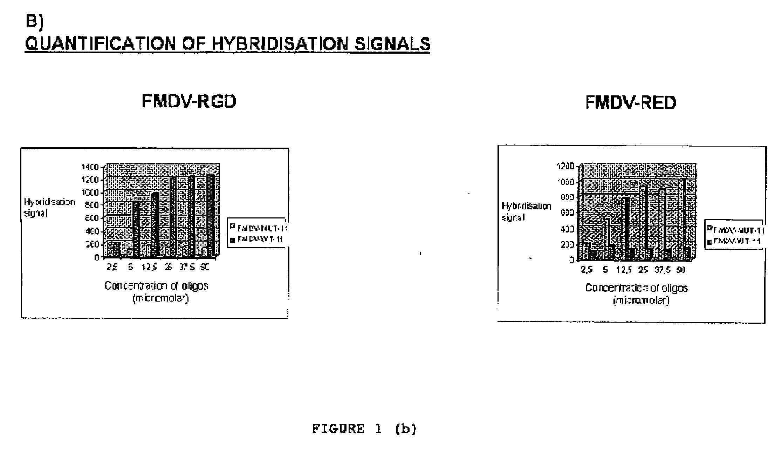

Figure 1.-A) Description of the hybridisation signals obtained by DNA microchip with the genomes FMDV-RGD and FMDV-RED for different concentrations of the oligonucleotides FMDV-WT-11 and FMDV-MUT-11. Hyb. T: Hybridisation temperature. Wash. T: Washing temperature. The higher values: 2.5, 5, 12.5, 25, 37.5 and 50 represent the concentrations (in micromolars) of the oligonucleotides deposited. **B)** Quantification of hybridisation signals obtained at the different points for each of the genomes FMDV-RGD and FMDV-RED. The hybridisation signals have been analysed using the Scanner model GMS 418 array scanner from Genetic Microsystems. Quantification of the signals (the mean value of the two values for each duplicate is always taken) was done with the statistical package "Statistics" of the software "Array Scanner" provided with the kit.

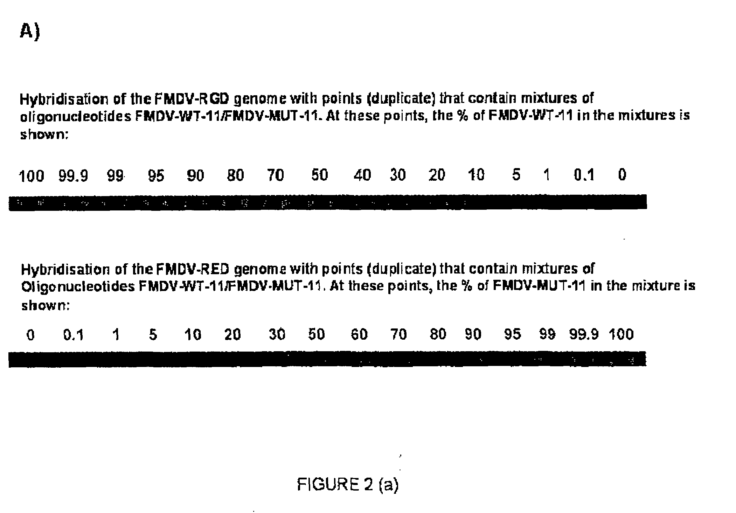

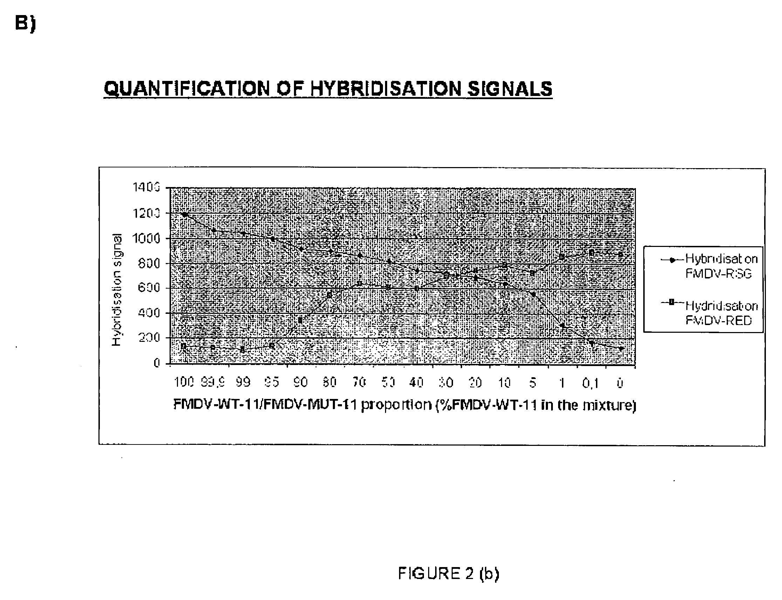

Figure 2.-A) Description of the hybridisation signals obtained using a DNA microchip with the FMDV-RGD and FMV-RED genomes, at a series of points (duplicates) that contain mixtures of the oligonucleotides FMDV-WT-11 and FMDV-MUT-11 in different relative proportions (between 100% and 0%). The higher values indicate in each case the percentage of oligonucleotides FMDV-WT-11 or FMDV-MUT-11 in the mixture. B) Quantification curve of the hybridisation signals obtained. The hybridisation signals have been analysed and quantified as indicated in the description of figure 1B.

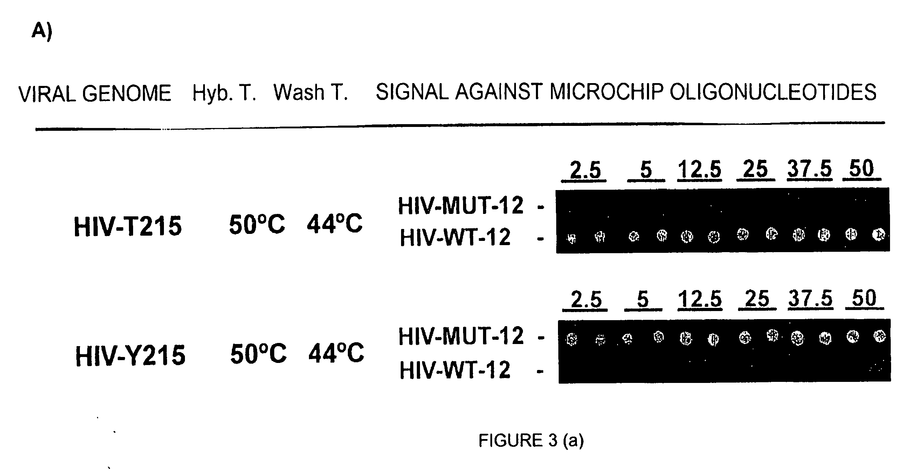

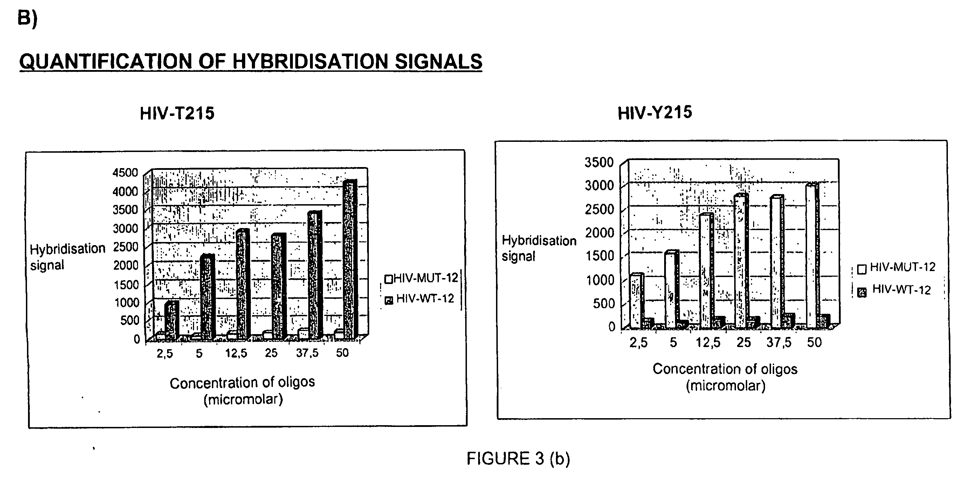

Figure 3.-A) Description of the hybridisation signals obtained using a DNA microchip with the genomes HIV-T215 and HIV-Y215 for different concentrations of the oligonucleotides HIV-WT-12 and HIV-MUT-12. Hyb. T.: Hybridisation temperature; Wash. T.: Washing temperature. Higher values: 2.5, 5, 12.5, 25, 37.5, and 50 represent the concentrations (micromolar) of the oligonucleotides deposited. B). Quantification of hybridisation signals obtained at the different points for each of the genomes HIV-T215 and HIV-Y215. The hybridisation signals have been analysed and quantified as described in figure 1B.

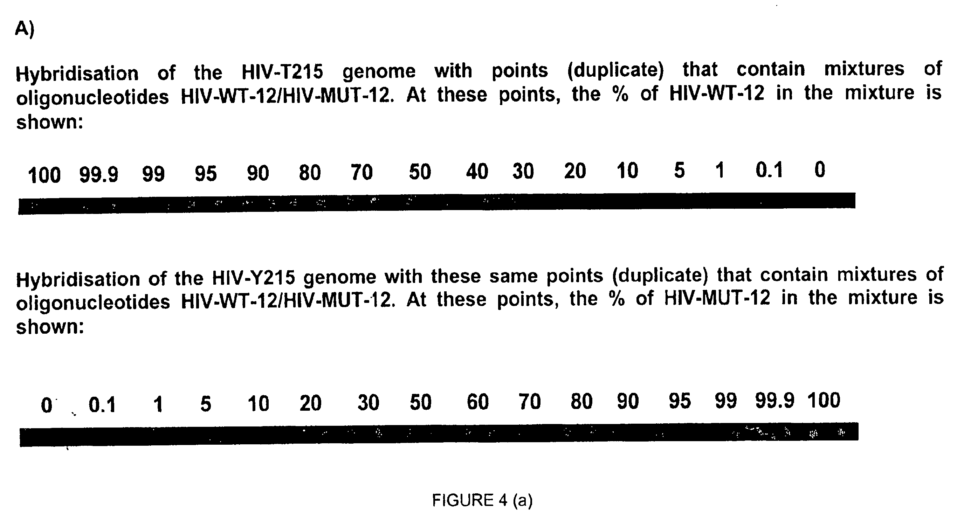

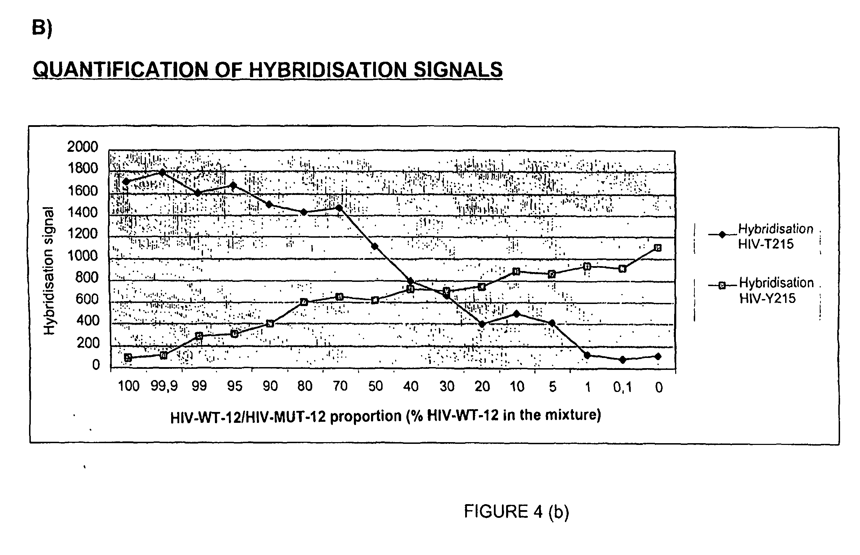

Figure 4. A) Description of the hybridisation signals obtained using a DNA microchip with the genomes HIV-T215 and HIV-Y215 in a series of points (duplicates) that contain mixtures of the oligonucleotides HIV-WT-12 and HIV-MUT-12 in different relative proportions (between 100% and 0%). The higher values in each case represent the percentage of oligonucleotide HIV-WT-12 or HIV-MUT-12 in the mixture. B). Quantification curve of the hybridisation signals obtained. The hybridisation signals have been analysed and quantified as indicated in the description of figure 1B.

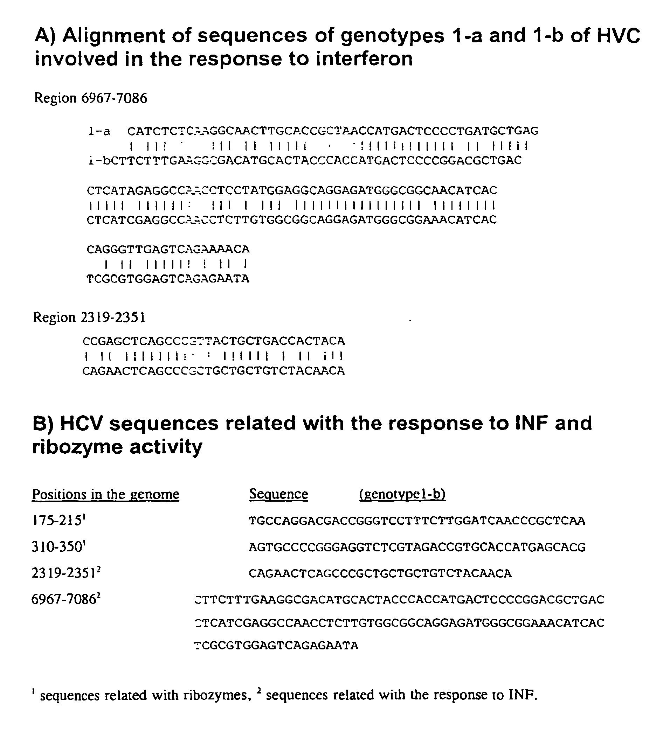

Figure 5.- Description of the HCV sequences involved in the response to interferon and with ribozyme activity. A) Alignment of ribozyme sequences of genotypes 1-a and 1-b of HCV involved in the response to interferon. B) HCV sequences related with the response to INF and ribozyme activity.

### EXAMPLES OF EMBODIMENTS OF THE INVENTION

The following examples are illustrative and should not be considered as limiting the scope of the invention.

### Example 1. -Detection and characterisation of minority genomes of the foot-and-mouth disease virus (FMDV).

As a test of the present original invention, DNA microchips have been constructed that contain oligonucleotides specific to the region that lies between amino acids 138 and 148 of protein VP1 of the viral capsid (nucleotide positions 3609 to 3651 of the FMDV genome).

The microchips constructed have the following characteristics:
- They have synthesized 4 oligonucleotides of 11 and 15 nucleotides long (nt), all these have an interrogant position in the central nucleotide and the flanking sequences identical to the original viral genome. The oligonucleotides have been chemically bound to a primary amine ("C6 aminolinker", also called "C6") at its 5' end so that this can react with free aldehyde groups produced by the previous treatment of the glass. Between the primary amine and the sequence of 11 or 15nt there is a 15 thymidine spacer (T₁₅) to facilitate hybridisation. The sequence of the four oligonucleotides used is as follows:
   - "FMDV-WT-15": 5'-C6-T₁₅CAAATCCCCGCGTGC-3'
   - "FMDV-MUT-15": 5'-C6-T₁₅CAAATCCTCGCGTGC-3'
   - "FMDV-WT-11": 5'-C6-T₁₅AATCCCCGCGT-3'
   - "FMDV-MUT-11": 5'-C6-T₁₅AATCCTCGCGT-3'
- The presynthesized oligonucleotides are immobilized in predetermined positions on the glass slides by an automatic system ("DNA arrayer").
- Each of the four oligonucleotides has been deposited in duplicate in the six points of the microchip at final concentrations of 2.5, 5, 12.5, 25, 37.5 and 50µM (micromolar), respectively.
- Moreover, spots are also deposited in duplicate of the mixtures FMV-WT-15/FMV -MUT-15 and FMV-MT-11/FMV-MUT 11 in the following proportions: 100:0, 99.9:0.1, 99:1, 95:5, 90:10, 80:20, 70:30, 60:40, 50:50, 40: 60, 30: 70, 20:80, 10:90, 5:95, 1:99, 0.1:99.9, 0:100. This permits the following:

- To determine the %age minimum detectable amount of the minority genome
- To draw up quantification curves
- Two 31nt oligonucleotides identical to the wild type virus genome ("FMV-RGD") or mutant ("FMV-RED"), labelled with the fluorochrome "Cy3" in its 5' end. The sequence of this is as follows:
   "FMV-RGD": 5'-Cy3-CCGCCAGTGCACGCGGGGATTTGGCTCACCT-3'
   "FMV-RED": 5'-Cy3-CCGCCAGTGCACGCGAGGATTTGGCTCACCT-3'
The results are described in Figures 1 and 2. From figure 1 it is clear that the use of DNA microchips permits hybridisation of the specific oligonucleotide from a concentration of 5 µM.

Figure 2 shows a curve for quantification of the hybridisation signals obtained at the different points with mixtures of oligonucleotides for each of the genomes FMV-RGD and FMV-RED. From the point of inflexion of these curves (that corresponds to a signal value of around 300 in both cases and that indicates the appearance of a specific signal above the baseline) it is clear that the minority oligonucleotide in the mixture can be detected even when this only corresponds to 1 to 5% of the mixture (hybridisation of FMV-RGD) or from 10-20% (hybridisation of FMV-RED).

### Example 2: Detection and characterisation of minority genomes of the human immunodeficiency virus (HIV) that are carriers of mutations for resistance to Zydovudine (AZT)

To test the present invention, DNA microchips have been constructed that contain specific oligonucleotides of HIV to detect the mutation T215Y of resistance to AZT. These oligonucleotides are complementary to the region that lies between amino acids 210 and 220 of the reverse transcriptase of HIV-1 (nucleotide positions 3179 to 3211 of the isolate CAM-1 of HIV-1).

The microchips constructed have the following characteristics:
- 4 oligonucleotides of between 12 and 16 oligonucleotides (nt) long have been synthesized all with the two adjacent interrogant positions (ACC→TAC) in its centre and identical flanking sequences to the viral genome. The oligonucleotides have been chemically bound to a primary amine ("C6 aminolinker", also called "C6") at its 5'end, so that it can react with the free aldehyde groups that are produced by the previous treatment of the glass. Between the primary amine and the sequence of 12 or 16nt there is a 15 thymidine spacer (T₁₅) to facilitate hybridisation. The sequences of the four oligonucleotides used are as follows:
   - "HIV-WT-16": 5'-C6-T₁₅TGGTGTGGTAAGTCCC-3'
   - "HIV-MUT-16": 5' -C6-T₁₅TGGTGTGTAAAGTCCC-3'
   - "HIV-WT-12": 5'-C6-T₁₅GTGTGGTAAGTC-3'
   - "HIV-MUT-12": 5'-C6-T₁₅GTGTGTAAAGTC-3'
- The presynthesized oligonucleotides are immobilised in predetermined positions on the glass slides by an automated system ("DNA arrayer").
- Each of the four oligonucleotides has been deposited in duplicate on the six points of the microchip at final concentrations of 2.5, 5, 12.5, 25, 37.5 and 50 µM, respectively.
- Moreover, spots are also deposited in duplicate of the mixtures HIV-WT-16/HIV-MUT-16 and HIV-WT-12/HIV-MUT-12 in the following proportions:
   100:0, 99.9:0.1, 99:1, 95:5, 90:10, 80:20, 70:30, 60:40, 50:50, 40: 60, 30: 70, 20:80, 10:90, 5:95, 1:99, 0.1:99.9, 0:100. This permits the following:
      -To determine the %age minimum detectable of the minority genome
      - To draw up quantification curves.
- Two 32 nt oligonucleotides identical to the wild type genome of the virus ("HIV-T215") or to the mutant genome ("HIV-Y215") were used, labelled with fluorochrome "Cy3" at its 5'end. Its sequence is as follows:
   - "HIV-T215": 5'-Cy3-
      TTGAGGTGGGGACTTACCACACCAGACAAAAA-3'
   - "HIV-Y215" : 5'-Cy3-
      TTGAGGTGGGGACTTTACACACCAGACAAAAA-3'

The results are described in figures 3 and 4. From figure 3 it can be seen that, in this case, the use of DNA microchips permits hybridisation of the specific oligonucleotide from a concentration of 2.5µM. Likewise, it can be seen that the degree of specificity reached is very high and the non-specific hybridisations are left with a signal that is not distinguishable from the background (always less than 200 units), compared to the 800 or more units obtained by specific hybridisation to oligonucleotides with a concentration over 2.5µM.

Figure 4 shows a quantification curve of the hybridisation signals obtained at the different points with oligonucleotide mixtures, for each of the genomes HIV-T215 and HIV-Y215. The point of inflexion of the curves (that corresponds to a signal value of around 300 in both cases and that indicates appearance of a specific signal above the background) demonstrates the possibility of detecting the minority oligonucleotide in the mixture even when this is present from 1 to 5% in both cases (hybridisation of HIV-T215 and HIV-Y215).

### EXAMPLE 3.-Detection and characterisation of memory genomes in populations of the foot-and-mouth disease virus (FMV)

### Example 3.1-Detection method for the memory genome "RED" of FMV resistant to a monoclonal antibody.

Starting with a mutant of FMV that contains the sequence Arg-Glu-Asp (Arginine-Glutamic-Aspartic, from hereon referred to as RED) in positions 141-143 of the protein VP1 of the viral capsid. This mutant was obtained by isolating mutants resistant to neutralization (loss of infectivity) of a population of FMV that contains the wild type sequence Arg-Gly-Asp (arginine-glycine-aspartic, from hereon referred to as RGD) in positions 141-143 of protein VP1 of the viral capsid (positions 3628-3636 of the viral genome, according to the numeration described in Escarmis et al., 1999). Both the mutant with RED (from hereon referred to as FMV RED) and the parental virus RGD (from hereon referred to as FMV RGD) and also the monoclonal antibody SD6 (from hereon MA SD6) used in the selection of FMV RED from FMV RDG has been described previously (Martinez et a., 1997; Mateu et al., 1987; Mateu et al., 1989; Ruiz-Jarabo et al., 1999). After propagation in triplicate of samples of FMV RED in BHK-21 cells (cell line established from cells of hamster kidney, described in Stocker and Macpherson, 1964) it was observed that the RED sequence reverted to RGD as the virus multiplied. Given that FMV RED was initially an isolated clone of a viral plaque (originating from a single genome), the transition from FMV RED to FMV RDG had to result from a true reversion, i.e. from the nucleotide change A425→G (the number indicates the position in the encoding region of VP1, that is equivalent to position 3632 of the complete genome of FMV, according to the numeration described by Escarmis et al., 1999). Propagation of the FMV RED in triplicate consisted in the infection of 4x10⁶ BHK-21 cells in 4x10⁵ infectious plaque formed units (from hereon PFU) of the FMV RED. The serial infection process was repeated 25 times (or with 25 passages); in each passage 4x10⁶ BHK-21 cells were infected with 4x10⁵ PFU of the virus obtained in the previous infection (passage). The proportion of FMV RGD and FMV RED in the passages was determined by sequenciation of the genomes present in the population in the region of RED encoding for amino acids 141 to 143 of the protein VP1 and the surrounding region. The methods used for this analysis have been described previously (Ecarmis et al., 1996; Escarmis et al., 1999; Baranowski et al., 1998). After 10 passages in the conditions mentioned, the presence of genomic sequences encoding for RED was not detected in the consensus or average sequence (obtained by sequencing the genome population present in the sample analysed without using any previous molecular cloning process) of the population. We wanted to determine whether in passages 15 and 25 the population maintained in its mutant spectrum a molecular memory of its origin as FMV RED. To do this the frequency of mutations resistant to MA SD6 was determined and the sequence of several of these mutants in the region that encode positions 141 to 143 of VP1. For the three populations of the 15^{th} passage the frequencies were: 1.8 x 10⁻² , 1.4x10⁻² and 1.3 x 10⁻², respectively; and for the three populations of passage 25 the frequencies were 1.6 x 10⁻², 5.3x10⁻¹ and 2.7 x 10⁻⁴, respectively. For two control populations (the same FMV parent and other clonal population of the same lineage) the found frequencies were (4.1Γ0.5)x10⁻³ and 5.0Γ1.6)x10⁻⁴, respectively. The most conclusive evidence for the presence of memory was obtained by sequencing 15 clones of the population reverting from passages 15 and 15 clones reverting from passage 25, resistant to MA SD6. All the mutants analysed (30 of the 30 analysed) had the encoding genomic nucleotide sequence for RED whereas in the control populations very few mutants resistant to the monoclonal antibody SD6 included RED (only 4 of the 112 clones analysed) (P<0.001; Chi² test). Hence, the revertant population of FMV RGD maintained a stable memory of the anterior dominance of the virus with RED in the history of the virus. As additional evidence that the memory of the quasispecies is represented in minority components of mutant spectra, the revertant FMV RDG populations of passage 15 were passed in BHK21 cells 10 more times using between 10 and 100 PFUs to infect 10⁶ BHK-21 cells per passage resulting in loss of the memory genomes. When sequencing 15 mutants resistant to the monoclonal antibody, none of these showed the RED sequence although amino acid substitutions appeared in other positions (amino acids 139, 142, 143, 144, 146). The result demonstrates that memory of the quasispecies is a property of the mutant spectra taken together and not of individual genomes that make up the quasispecies.

The design of DNA microchips with specific oligonucleotides of the region lying between amino acids 138 and 148 of protein VP1 of the viral capsid (positions 3609-3651 of the FMV genome) permits the presence of RED minority memory genome to be detected in a viral quasispecies dominated by the RGD genome and to quantify the proportion in which it is present in different experimental conditions. Likewise, this also permits other nucleotide changes in the flanking regions to be detected. To do this a collection of 20 nt nucleotides is designed that each have, as a central interrogant position, each of the four possible nucleotides (A,C,G and T) in each of the positions from positions 3609-3651 of the FMV genome, according to the methods indicated in the detailed description of the invention. In all cases, the flanking regions correspond to the wild type genome sequence (RGD) in this region.

### Example 3.2. -Determination of memory genomes of the FMV virus with poly-A tails of variable length between positions 1119-1123 of the viral genome.

Additional evidence of the presence of memory in quasispecies of FMV was obtained by using the clone C₂₂⁹, greatly weakened by successive passages from plaque to plaque of the FMV as described in Escarmis et al., 1996. A unique characteristic of this clone not found in any of the natural or laboratory isolates of FMV, is the presence of a polyadenylate portion (or poly A, section of polymerised AMP) of heterogeneous length with a mean of around 23 residues of adenylic acid (abbreviated A). This poly A is situated in the region of the genome that precedes the second triplet of AUG that functions in initiation of protein synthesis (the polyprotein) encoded by the genome of FMV (Escarmis et al., 1996). When clone C₂₂⁹ was propagated in cell cultures using large populations (in each passage 4 x 10⁶ BHK-21 cells were infected with 10⁶ to 10⁷ PFUs of the virus obtained in the previous passage) there was an increase in the replicative efficacy of the virus (Escarmis et al., 1999). In this process of gain in replicative efficacy, the first molecular change observed was the loss of polyadenylate that was not detectable in passage 20 (Escarmis et al., 1999). When the population of clone C₂₂⁹ passed 50 times in BHK-21 cells were then molecularly and biologically cloned, genomes were detected with a greater number of A residues than the number present in the wild type FMV, preceding the second functional AUG triplet. A greater number of As were detected in 8 of the 70 clones analysed whereas no genome was detected with a greater number of As in 40 clones of a control population of FMV submitted to the same number of passages but that originates in a clone without additional As (0.01>P>0.0025; Chi²). In other words, the FMV C₂₂⁹ maintained a memory of its previous history in the form of minority genomes of the quasispecies. These examples with FMV prove the existence of a molecular memory in FMV populations that reveals the previous evolutionary history of the virus.

### Example 4.- Method for the detection of memory genomes of the human immunodeficiency virus (HIV) that are carriers of the mutations that confer resistance to drugs in treated patients.

The specific DNA chips to detect memory genomes of the human immunodeficiency virus (HIV) contain a collection of oligonucleotides with interrogant positions between the wild type or mutant strains are recorded in Table II. The DNA chip also includes, in addition to the nucleotides that include the 362 interrogant positions listed in the table (181 corresponding to wild type virus and another 181 corresponding to the resistance mutants), all the possible individual nucleotide variants of each of the codons in which a resistance mutation (10 oligonucleotides per position, according to Table I). The flanking sequences necessary have been designed both for wild type and mutant oligonucleotides as a function of their total sequence homology with the HXB2 strain of HIV-1, subtype B (Ratner et al., 1985; accession no. in the genbank database KO3455).

These flanking sequences vary in relation to the type of DNA chip designed. In the case of using MO1, the flanking sequences are comprised of between 5 and 50 nucleotides on each side. If the hybridisation strategy of stacking of bases is used, the discriminatory position is at the 5'end of an oligonucleotide of between 5 and 100 nucleotides long. To detect mutant genomes with insertions of one or two amino acids mutant oligonucleotides are designed that contain the three or six inserted nucleotides as indicated in Table II.

On the other hand, the sequence context in which the interrogant position is found is variable in relation to the genetic diversity of HIV. Because of this heterogeneity, HIV is classified into two different species (HIV-1 and HIV-2) into groups (M, O and N for HIV-1) and into subtypes (A-J for HIV-1 group M), each with a different geographical distribution. Because of this, oligonucleotides are designed with flanking sequences that correspond to different subtypes of B of the HIV-1 group M (McCutchan et al., 1996; Gao et al., 1998; Paraskevis et al., 1999), of the HIV-1 group O (Janssens et al, 1999; Mas et al., 1999), to the HIV-1 group N (Simon etc al., 1998) and to HIV-2 (Clavel et al., 1986; Chen et al., 1997). To analyse inter-subtype or intergroup recombinant virus, oligonucleotides designed in relation to the sequence that the recombinant has in the PR and RT regions of the pol gene are used (Robertson et al., 1995; Takeshi et al., 1999).

Another reason for which a continuous update of the oligonucleotide sequences is required is because of the constant description of new resistance mutations in response to treatment with new drugs or to new combined therapies (Menendez Arias et al., 1998; Winters et al., 1998; Schinazi et al., 1999; Briones et al., 2000). The description of new mutations is also occasionally due to the analysis of databases that correlate patterns of genotype and phenotype resistance (Hertogs et al., 2000).

Because of all this, owing to the constant detection of genetic variants of HIV that differ to a greater or lesser extent from known sequences, the oligonucleotide catalogues used need to be continually updated in relation to the descriptions and sequences published and/or entered in the database (Korber B et al., 1998; regular update in http://hiv-web.lanl.gov).

**Table 2**

| **List of resistance mutations of the Human Immunodeficiency Virus (HIV) to reverse transcriptase (RT) inhibitors and the protease (PR)** | | | | | |
|---|---|---|---|---|---|
| A) MUTATIONS ASSOCIATED WITH NUCLEOSIDE ANALOGS OF RT INHIBITORS (NRTI) | | | | | |
| N1 ID: Mutation Appears as mutation Observations | | | | | |
| | *wt* | *mut* | Individual | Combined | |
| 1:M41L | ATG | TTG | + | + | M.1.(AZT) |
| 2:M41L | ATG | CTG | + | + | M.1. (AZT) Rare |
| 3: E44D | GAA | GAT | - | + | Rare |
| 4: E44A | GAA | GCA | - | + | Rare |
| 5: I50T | ATC | ACC | - | + | Rare |
| 6: A62V | GCT | GTT | - | + | M.2 to Q151M (MDR) |
| 7: K65R | AAA | AGA | + | + | M.1 (ADV) |
| 8: D67G | GAC | GGC | - | + | Rare |
| 9: D67N | GAC | AAC | - | + | |
| 10: T69A | ACT | GCT | - | + | |
| 11: T69D | ACT | GAT | + | + | M.1 (DDC) |
| 12: T69S | ACT | AGT | - | + | M. before insertion of 2aa |
| 13-17: 69-ss-70 | - | i:AGTAGT | | | |
| | | i. AGTTCT | | | |
| | | i. AGCAGT | | | |
| | | i. AGCTCT | | | |
| | | i. TCTAGT | | | |
| 18: 69.SG-70 | - | i. AGTGGT | - | + | Ins. of 2 aa (MDR) |
| 19-20: | - | i: AGTGCT | - | + | Ins. of 2 aa (MDR) |
| 69-SA-70 | - | i. AGCGCT | - | + | |
| 21: 69-ST-70 | | i. TCTACC | - | + | Ins. of 2 aa:rare (MDR) |
| 22: 69-SV-70 | | i. AGCGTG | - | + | Ins. of 2 aa:rare (MDR) |
| 23: 69-AG-70 | | i. GCTGGT | - | + | Ins. of 2 aa:rare (MDR) |
| 24: 69-EA-70 | | i. GAAGCA | - | + | Ins. of 2 aa:rare (MDR) |
| 25: 69-EE-70 | | i. GAAGAA | - | + | Ins. of 2 aa:rare (MDR) |
| 26: 69-MT-70 | | i. ATGACC | - | + | Ins. of 2 aa:rare (MDR) |
| 27: 69-TS-70 | | i. ACCTCT | - | + | Ins. of 2 aa:rare (MDR) |
| 28: 69-VG-70 | | i. GTGGGT | - | + | Ins. of 2 aa:rare (MDR) |
| 29: 69-D-70 | | i. GAT | + | + | Ins. of 1 aa:rare (MDR) |
| 30: K70E | AAA | GAA | - | + | M.1. (AZT); high polmorph. |
| 31: K70N | AAA | AAT | - | + | M.1. (AZT); high polmorph |
| 32: K70N | AAA | AAC | + | + | M.1. (AZT); high polmorph |
| 33:K70R | AAA | AGA | + | + | M1. (AZT); high polymorph. |
| 34: L74V | TTA | GTA | - | + | M.1 (ddI) |
| 35: V75I | GTA | ATA | + | + | M. 2 to Q151M (MDR) |
| 36: V75T | GTA | ACA | + | + | M.1 (d4T) |
| 37: F77L | TTC | CTC | - | + | M2 to Q151M (MDR) |
| 38: E89G | GAA | GGA | - | + | Rare |
| 39: V90I | GTT | ATT | - | + | Rare |
| 40: A114S | GCT | AGT | - | + | Rare |
| 41: Y115F | TAT | TTT | + | + | Rare |
| 42: F116Y | TTC | TAC | - | + | M2 to Q151M (MDR) |
| 43: V118I | GTA | ATA | - | + | Rare |
| 44: P119S | CCC | TCC | - | + | Rare |
| 45: Q151M | CAG | ATG | + | + | MDR |
| 46: P157S | CCG | TCG | + | + | |
| 47: R172K | AGA | AAA | - | + | Rare |
| 48: I178M | ATA | ATG | - | + | Rare |
| 49: V179D | GTT | GAT | - | + | Rare |
| 50: M184I | ATG | ATA | + | + | |
| 51: M184T | ATG | ACG | + | + | |
| 52: M184V | ATG | GTG | + | + | M.1. (3TC,ABC) |
| 53: L210W | TTG | TGG | - | + | |
| 54: R211K | CGA | AAA | - | + | |
| 55. T215C | ACC | TGC | - | + | M.1.(AZT); Rare |
| 56: T215F | ACC | TTC | + | + | M.1 (AZT) |
| 57: T215S | ACT | TCT | - | + | M1 (AZT); Rare |
| 58: T215Y | ACC | TAC | + | + | M1 (AZT) |
| 59: K219E | AAA | GAA | - | + | Rare |
| 60:K219Q | AAA | CAA | - | + | Rare |
| 61: G333E | GGG | GAG | - | + | Rare |

| B) MUTATIONS ASSOCIATED WITH NON-NUCLEOSIDE ANALOGUES OF RT INHIBITORS (NNRTI) | | | | | |
|---|---|---|---|---|---|
| N1 ID: | Mutation | | Appears as mutation | | Observations |
| | *Wt* | Mut | Individual | Combined | Observations |
| 1:E6K | GAG | AAG | - | + | Rare |
| 2: L74I | TTA | ATA | - | + | Rare |
| 3: L74V | TTA | GTA | - | + | Rare |
| 4:V75I | GTA | ATA | - | + | Rare |
| 5: V75L | GTA | TTA | - | + | Rare |
| 6: V90I | GTA | ATA | - | + | Rare |
| 7: A98G | GCA | GGA | + | + | |
| 8: L100I | TTA | ATA | + | + | |
| 9: L100I | CTA | ATA | - | + | Less frequent than previous |
| 10: L100I | TTG | ATA | - | + | Less frequent than previous |
| 11: K101A | AAA | GCA | - | + | Rare |
| 12:K101E | AAA | GAA | + | + | Rare |
| 13:K101I | AAA | ATA | - | + | Rare |
| 14: K101Q | AAA | CAA | - | + | Rare |
| 15: K103N | AAA | AAC | + | + | M.1 (NVP,EFV,DLV) |
| 16: K103N | AAG | AAT | + | + | M1 (NVP,EFV,DLV) |
| 17: K103Q | AAA | CAA | - | + | Rare |
| 18: K103R | AAA | AGA | - | + | Rare |
| 19: K103T | AAA | ACA | + | + | |
| 20: V106A | GTA | GCA | + | + | |
| 21: L106I | GTA | ATA | - | + | Rare |
| 22:V106L | GTA | TTA | - | + | Rare |
| 23:V108I | GTA | ATA | + | + | |
| 24: V108I | GTT | ATT | + | + | Less frequent than previous |
| 25: E138G | GAG | GGG | - | + | Rare |
| 26: E138K | GAG | AAG | - | + | Rare |
| 27: E138R | GAG | AGG | - | + | Rare |
| 28: T139I | ACA | ATA | - | + | Rare |
| 29: G141E | GGG | GAG | - | + | Rare |
| 30: V179D | GTT | GAT | + | + | |
| 31: V179E | GTT | GAG | - | + | Rare |
| 32: Y181C | TAT | TGT | + | + | M.1 (NVP,DLV) |
| 33: Y181H | TAT | CAT | - | + | Rare |
| 34: Y181I | TAT | ATT | + | + | M.1.(NVP,DLV); rare |
| 35: Y181L | TAT | CTT | - | + | Rare |
| 36: Y188C | TAT | TGT | + | + | |
| 37: Y188H | TAT | CAT | - | + | Rare |
| 38: Y188L | TAT | TTA | + | + | |
| 39: Y188L | TAT | CTT | - | + | Rare |
| 40: V189I | GTA | ATA | - | + | Rare |
| 41: G190A | GGA | GCA | + | + | |
| 42: G190E | GGA | GAA | - | + | Rare |
| 43: G190Q | GGA | CAA | - | + | Rare |
| 44: G190S | GGA | AGC | + | + | |
| 45: G190T | GGA | ACA | - | + | Rare |
| 46: P225H | CCT | CAT | + | + | |
| 47: F227L | TTC | TTA | - | + | Rare |
| 48: F227L | TTC | TTG | - | + | Rare |
| 49:F227L | TTC | CTC | - | + | Rare |
| 50: M230L | ATG | TTG | - | + | Rare |
| 51:E233V | GAA | GTA | - | + | Rare |
| 52: L234I | CTC | ATC | - | + | Rare |
| 53:P236L | CCT | CTT | + | + | |
| 54: K238T | AAA | ACA | - | + | Rare |

| C) MUTATIONS ASSOCIATED WITH PROTEASE INHIBITORS (PI) | | | | | |
|---|---|---|---|---|---|
| N1 ID: | Mutation | Appears as mutation | | | |
| Observations | | | | | |
| 1: R8Q | CGA | CAA | + | + | |
| 2: R8K | CGA | AAA | - | + | Rare |
| 3: L10F | CTC | CGC | - | + | |
| 4: L10F | CTC | TTC | - | + | Less frequent than previous |
| 5: L10I | CTC | ATC | - | + | |
| 6: L10R | CTC | CGC | - | + | |
| 7: L10V | CTA | GTA | - | + | Rare |
| 8: L10Y | AAA | TAT | - | + | Rare |
| 9: L10Y | AAA | TAC | - | + | Rare |
| 10: L11V | ATA | GTA | - | + | Rare |
| 11: L13V | ATA | GTA | - | + | |
| 12: K20M | AAG | ATG | - | + | |
| 13: K20R | AAG | AGG | - | + | |
| 14: L23I | CTA | ATA | - | + | Rare |
| 15: L23V | TTA | GTA | - | + | |
| 16: L24I | TTA | ATA | - | + | |
| 17: L24V | TTA | GTA | - | + | Rare |
| 18: D30N | GAT | AAT | + | + | M.1 (NFV) |
| 19: V32I | GTA | ATA | - | + | |
| 20: L33F | TTA | TTT | - | + | |
| 21:E34K | GAA | AAA | - | + | Rare |
| 22:E34V | GAA | GTA | - | + | Rare |
| 23: E35D | GAA | GAT | - | + | |
| 24: M36I | ATG | ATA | - | + | M.2 very frequent |
| 25: K45E | AAA | GAA | - | + | Rare |
| 26: K45I | AAA | ATA | - | + | Rare |
| 27: M46F | ATG | TTC | - | + | Rare |
| 28: M46I | ATG | ATA | + | + | M.1 (IDV) |
| 29: M46L | ATG | TTG | + | + | M.1 (IDV) |
| 30: M46V | ATG | GTG | - | + | Rare |
| 31: I47A | ATA | GCA | - | + | Rare |
| 32: I47V | ATA | GTA | - | + | |
| 33: G48V | GGG | GTG | + | + | M.1 (SQV) |
| 34: I50L | ATC | TTA | - | + | Rare |
| 35: I50L | ATC | CTC | - | + | Rare |
| 36: I50V | ATT | GTT | + | + | M.1. (APV) |
| 37: I54L | ATC | TTA | - | + | Rare |
| 38: I54L | ATC | CTC | - | + | Rare |
| 39: I54M | ATC | ATG | - | + | |
| 40: I54V | ATC | GTC | - | + | M.2.very frequent |
| 41: D60E | GAT | GAA | - | + | Rare |
| 42: L63P | CTC | CCC | - | + | Natural high polymorphism |
| 43: L63Q | CTG | CAG | - | + | Natural high polymorphism |
| 44: L63V | TTA | GTA | - | + | Natural high polymorphism |
| 45: A71T | GCT | ACT | - | + | |
| 46: A71V | GCT | GTT | - | + | M.2.very frequent |
| 47: G73S | GGT | GCT | - | + | |
| 48: G73S | GGT | AGT | - | + | Less frequent than previous |
| 49: V75I | GTC | ATC | - | + | Rare |
| 50: L76M | TTG | ATG | - | + | Rare |
| 51: V77I | GTC | ATC | - | + | |
| 52:P81T | CCT | ACT | - | + | Rare |
| 53: V82A | GTC | GCC | + | + | M.1 (IDV, RTV) |
| 54: V82F | GTC | TTC | + | + | M.1. (IDV, RTV |
| 55: V82I | GTC | ATC | + | + | M.1. (IDV) |
| 56: V82S | GTC | TCC | + | + | M.1. (RTV) |
| 57: V82T | GTC | ACC | + | + | M.1. (IDV, RTV) |
| 58: I84A | ATA | GCA | - | + | Rare |
| 59: I84V | ATA | GTA | + | + | M.2. Very common |
| 60: N88D | AAT | GAT | - | + | |
| 61: N88S | AAT | AGT | - | + | |
| 62: L89M | TTG | ATG | - | + | |
| 63: L90I | TTA | ATA | - | + | Rare |
| 64: L90M | TTG | ATG | + | + | M.1 (SQV,NFV) |
| 65: T91S | ACT | TCT | - | + | Rare |
| 66: L97V | TTA | GTA | - | + | Rare |

| | | | | | |
|---|---|---|---|---|---|
| Legend for Table II 1. The resistance mutations have been divided with respect to the three families of antiretroviral drugs: (A) Nucleoside analogues of RT inhibitors, (B) Nucleoside non-analogues of RT inhibitors and (C) protease inhibitors (PI). These positions are those that have been described to be responsible for the resistance to different drugs. All these are included in the DNA microchip specific for HIV together with all the possible nucleotide variants of each of the codons studied. 2. The first column shows an identification number (N1 ID) and the amino acid change associated with the resistance. This shows the amino acid of the wild type strain (wt), the position this occupies in the gene and the amino acid of the mutant strain. Therefore, for example the change M41L in the (A) part of the Table corresponds to a change from leucine to methionine in position 41 of the RT gene. Mutations with identification number 13 to 29 of the section (A) correspond to insertions of the amino acids indicated between codons 69 and 70 of RT. 3. The second and third columns show the nucleotide sequence of the wild type (wt) and mutant (mut) strains. In the cases of insertion of amino acids, the column corresponding to the mutant genome shows the 3 or 6 inserted nucleotides. As a reference wild type genome the strain HXB2 of HIV-1 subtype B was used. 4. The sixth column includes a series of observations and additional data related to each mutation. M1 and M2 indicate primary or secondary mutations for the inhibitor, respectively, implying that these develop, sooner or later, as a response to treatment with this drug. It is also recorded whether the mutation appears only rarely in treated patients (rare), whether this consists of an amino acid insertion (Ins.) or whether this position is especially variable in HIV-1, therefore presenting a natural polymorphism. In the cases in which the drug responsible for each mutation is indicated in parentheses the following abbreviations have been used: AZT, zidovudine, ddI, didanosine, ddC, zalcitabine; 3TC, lamivudine; d4T, estavudine; ABC, abacivir; ADV, adefovir; NVP, neviparin; EFV, efavirenz; DLV, delavirdin; SQV, saquinavir; RTV, ritonavir; IDV, indinavir; NFV, nelfinivir; APV, amprenavir. In part (A) the indication MDR refers to mutations associated with multiresistance to different drugs of the same family. 5. The data compiled in Table II proceed from Antoni et al., 1997; Winters et al., 1998; Menendez-Arias et al., 1998; Schinazi et al., 1999; De Jong et al., 1999; Korber et al., 1998; Briones et al., 2000; and Hertogs et al., 2000. | | | | | |

A DNA microchip was constructed with type 1 memory oligonucleotides with a length of 15 nt, complementary to the region of the viral genome that includes the codons listed in Table II. All of these belong to the protease gene (PR) or the reverse transcriptase gene (RT) of HIV-1 HXB2 of subtype B. In this genome type, fragments of the protease (PR) and the reverse transcriptase (RT) of the pol gene correspond, respectively, to positions 2253-2549 and 2550-4229.
Additional negative controls were also taken as universal oligonucleotides of pUC18 (Sambrook et al., 1989) and others that they did not expect to find in the viral population as negative controls. The procedure was as follows:

### 1. RNA viral extraction and cDNA synthesis

The blood samples were extracted from HIV-positive patients in 10 ml tubes with EDTA. Plasma separation was done from total blood by centrifugation at 5000 g for 20 minutes. With this an upper phase that contained the plasma was obtained, a lower phase (hematocrit) and an interphase that contained peripheral blood lymphocytes (PBMCs).

From the 0.5-1 ml of plasma obtained, the RNA of HIV was obtained by viral lysis with guanidine isothiocyanate, followed by adsorption to silica particles, washing and resuspension (Boom et al., 1990). An alterative method for RNA viral extraction consists in ultracentrifugation of the plasma at 23,000 g for 1 hour to obtain a sediment of viral particles, followed by lysis of the virus and resuspension of the RNA.

### 2. Extraction of proviral DNA.

In the cases in which proviral DNA integrated in the genome of peripheral blood lymphocytes (PBMCs) is analysed, after separation of the cells as described in point 1 DNA extraction is carried out. The system used consists of sedimentation of the PBMCs by centrifugation at 10,000g for 5 min, resuspension and cell lysis by proteinase k/Tween 20 (Innis et al., 1990).

### 3. Synthesis of cDNA

The synthesis of cDNA from the viral RNA obtained as indicated in point 1 is carried out as follows: 10 ml of RNA is incubated with 3400 ng of a specific inhibitor of the region that is going to be retrotranscribed (starter B for the RT or starter F for the PR, see points 4 and 5). After incubation at 70°C for 10 minutes, 8 ml of a mixture are added that contain: 10 units of AMV-RT (Promega, Madison, MI), 1ml of dNTPs 10mM mixture, 25 units of RNAsin, 4 ml of AMV-RT buffer 5x and 1.37 ml of water. Incubation is carried out at 42°C for one hour followed by another at 70°C for 5 minutes. The cDNA can be used directly to carry out PCR amplification or kept at 70°C until use.

### 4. Amplification of PCR from a fragment of RT

A fragment of RT of 647 bp (codons 19 to 234) is amplified by nested PCR. The first round of PCR is done using the direct initiator A and the reverse B:
A: 5'-GGTTGCACTTTAAATTTTCCCATTAGTCCTATT-3'
B: 5'-TACTAACTTCTGTATGTCATTGACAGTCCAGCT-3'

The second round of PCR is done using the initiators C and D:
C: 5'-GTTAAACAATGGCCATTGACAG-3'
D: 5'-AGTTCATAACCCATCCAAAGG-3'

The conditions used are:
a) for the first PCR: an initial denaturisation of 5 minutes at 94°C followed by 40 cycles of (94 c-30s/55 c-30s/72 c-1 minute) and a final elongation of 5 minutes at 72 C; and
b) for the second PCR: denaturisation of 1 minute at 94 C, 35 cycles of (94 c-30s/57 C-30 s/72 C-30 s) and final elongation of 5 minutes at 72 C.

### 5. PCR amplification of PR

Using nested PCR 401 base pairs were amplified that contain the total PR. The first round of PCR was done using the direct starter E and the reverse F:
E: 5'-GCCAACAGCCCCACCAGAAGAGAGC-3'
F: 5'-GGCCATTGTTTAACTTTTGGGCCATCC-3'

The second round of PCR was done using the starters G and H:
G: 5'- CAACTCCCTCTCAGAAGCAGGAGCCG-3'
H: 5'-CCATTCCTGGCTTTAATTTTACTGGTA-3'

The conditions used were:
a) or the first PCR: an initial denaturisation of 5 minutes at 94 C followed by 35 cycles of (94 C-30 s/56 c-30 s/72 C-1 minute) and a final elongation of 5 minutes at 72 C; and
b) for the second PCR the same conditions were used except that the elongation phase in the cycles is 54 C for 30 s

### 6. PCR amplification of complete PR and RT.

In a single process of nested PCR all the PR and RT regions of the pol gene can be amplified. Nevertheless, given that the fragment generated is very large (more than 2000 nucleotides) the efficiency of the amplification reduces notably compared to the PR and RT amplifications done separately. Given that the method described in this invention tries to find minority memory genomes in the quasispecies, it can serve to optimise the amplification efficiency. It is, therefore, preferable to carry out amplifications of the regions of interest as indicated in points 4 and 5.

### 7. Purification of fragments amplified by PCR and fluorescent labelling

The viral genome fragments amplified by PCR are isolated by agarose gel fractionation and purification in QIAquick columns (QIAquick PCR purification kit, Qiagen #28106). Approximately 1 µg of each of the purified fragments is mixed in a tube and fluorescently labelled in the following reaction mixture: 71µg of H₂O, 10µl of PCR buffer 10 x (500 mM KCl, 100 mM Tris-Cl pH 8.3, 15 mM MgCl₂, 0.1% gelatine), 10µl of dNTPs (2mM of each one), 5 µl Cy5-dCTP (1mM), 2µl (100 pmol/µl) of the oligonucleotides appropriate for RT and PR, 1µg (1-2ml) of the DNA mixture amplified by PCR, 1.0 µl of *Taq* DNA polymerase (5 units/µl). Single chain fluorescent cDNAs are generated by linear amplification of the template in accordance with the following conditions: denaturisation at 95 C for 2 minutes, amplification for 30 cycles of (94C-30s/55 C-30 s/72 C-30s), final extension at 72 C for 3 minutes. The fluorescent products are purified using a QIAquick (Qiagen) column and are resuspended in 50µl of 1x TE (10 mM Tris-Cl and 1 mM EDTA) pH 8.0, until a final concentration of approximately 20 ng/µl. For each 13 µl a total of 5.0 µl of SSC is added 20 [3 M NaCl, 0.3 M of sodium citrate (pH7.0)] and 2.0 µl 2% SDS is heated to 65 C for 30 s to dissolve the fluorescent sample and after centrifugation for 2 minutes at 5,000 g to eliminate sediments the supernatant is transferred to a new tube. The final concentration of the fluorescent mixture is approximately 0.15µg /µl in 20µl of 5XSSC and 0.2% of SDS.

Proceed in the same way for the labelling with a different fluorochrome (Cy3) of an amplified DNA fragment with the same oligonucleotides for RT and for PR of the HIV type virus.

### 8. Design and construction of memory oligonucleotides chip of HIV

The majority sequence in the RT and PR of the viral quasispecies extracted from a patient is determined by sequencing two chains of each fragment in the total viral quasispecies (consensus sequence), using the same oligonucleotides as for the internal amplification by nested PCR.

On a glass slide prepared with active aldehyde (CEL Associates) 600 MO1 oligonucleotides of 15 nt in length are printed, in which the central position (the eighth) is the interrogant position and, therefore, identifies the mutation in the sample marked fluorescently. In each point of the chip, 2 pmol (picomoles) of oligonucleotide in printing solution are placed and the oligonucleotides not incorporated are eliminated. The layout of the oligonucleotides in the chip is as follows:
a) a set of MO1 with an equal sequence to the HIV-1 genome, subtype B (Ratner et al. 1985, Genbank accession number KO3455) by duplicate, each oligonucleotide with 7 nucleotides flanking each side of each of the point mutations listed in Table II.
b) A set of MO1 with a sequence the same as the majority sequence found in a known sample sequence from a patient, also in duplicate. Again, the sequence of these MO1 includes 7 flanking nucleotides on each side of each of the point mutations listed in Table II. This set of oligonucleotides constitutes a positive control such that the intensity of the hybridisation signal identifies or is characteristic of the level of the majority genome;
c) A set of MO1 (in duplicate) of equal sequence to the set of section b) except for the interrogant position where now the base that identifies each of the point mutations listed in Table II is situated;
d) A series in quadruplicate of mixtures of different proportions of wild type (wt) and mutant (mut) oligonucleotides for amino acid 41 of RT (M41L). The proportions of the mutant in comparison to the wild type in the mixture are 1, 5x10^{-1,} 10¹, 10⁻², 10⁻³, 10⁻⁴, 10⁻⁵, 0. The graphical representation of the mean intensity of the hybridisation signal in each of these points permits us to construct a reference curve to quantify the hybridisation signals corresponding to memory genome mutations;
e) A series of 50 points containing the same amount of wt oligonucleotide for codon 41 of RT (ATG);
f) A series of 50 points containing the same amount of mut oligonucleotide for codon 41 of RT (ATG→TTG);
g) A series of 50 points containing the same amount of mut oligonucleotide for codon 41 of RT (ATG→CTG) ;
h) A series of 50 points containing the same amount of mut oligonucleotide for codon 41 of RT (ATG→GTG);
   The series e-h is controls that give us information about the intensity of the hybridisation signal for the wild type copy (e) and for the possible memory mutations (f-g). In turn, one or the three series of mutant oligonucleotides serve as a negative control since it is expected that its complementary sequence is not in the fluorescent mixture;
i) Two series of 10 points each containing the universal oligonucleotides of plasmid pUC18, that do not have complementary sequences to any oligonucleotide of HIV. These points serve as a negative control of 0% hybridisation.

### 9. Hybridisation of fluorescent samples with the microchip

Approximately 1/5th of the volume of the fluorescent mixtures are denaturised by boiling for 2 minutes and then placed in contact with the chip to permit hybridisation for 4 hours at 42 C. Elimination of the non-hybridised sample is done by two washes with SSC 2X at room temperature (22-25 C) for 5 minutes each and a third wash in the same conditions.

### 10. Microchip scanning

The availability of a scanner that permits the wet microchip to be read (without requiring previous drying) enabling us to assess the result of each wash and modify the conditions to maximise the ratio of signal/noise of the hybridisation. After finishing the washes, the microchip is left to air dry. Scanning is carried out with maximum values of the PMT (Photomultiplier tube) and laser that conserve the linearity and minimize the background noise.

### 11. Analysis of scanned images

The intensity of the fluorescence is quantified with Imagen software. There is a variety of software available free of charge on the internet. Nevertheless, as mentioned previously another aspect of the invention contemplates the development of new computer programmes that will increase the accuracy of the analyses.

As an example, we briefly describe the results obtained after applying the method of the invention to the clinical sample of a HIV-positive patient (see the legends of Table II for abbreviations of the drugs):
i) The patient had been receiving treatment with AZT+3TC+IDV for 8 months and therapeutic failure was detected on the basis of an increase in viral load to a value of 28,540 copies of HIV RNA per ml of blood plasma and reduced immunity determined on the basis of lymphocyte CD4+ count. Application of the method of the invention at this moment demonstrated the following mutations associated with resistance in the majority genome: M46L in the PR and M41L, M184V, L210W, T215Y in RT;
ii) At this moment the therapeutic regime was changed to the following combination: ddI+d4T+IDV that was maintained for 5 months until the second analysis of viral quasispecies genotype using the method of the invention. This detected the following mutations associated with resistance in the majority genome: M46L, L63P, A71V, I84V in the PR. Similarly, the following mutations associated with resistance were detected in the minority genome (in a proportion of 10⁻³ and 5x10⁻²): M46L and L63P in the PR; M41L and T215Y in the RT; and
iii) interpretation of these results indicates that after treatment with ddI+d4T+IDV, both mutations present in the RT in minority memory genomes (but not in the majority genome), have remained as memory mutations of genomes that had been majority genomes in the previous analysis. More specifically, these two mutations that determine resistance to AZT, were selected as majority genomes during the previous treatment (AZT+3TC+IDV) in response to one of the drugs of the therapeutic combination. After 5 months of treatment that did not include AZT, the resistance mutations to this drug remained only as memory genomes. The value of this invention lies in the fact that a physician unaware of the presence of these mutations in the memory genome could prescribe AZT in the new treatment if he did not detect mutations to these drugs in the majority genome. However, the presence of these mutations in the memory genomes of the quasispecies (as a result of a previous treatment that the physician may not know about) would mean that these would be rapidly selected for if the patient were exposed again to AZT resulting in rapid therapeutic failure in this patient.

### Example 5.-Method for the detection of memory genomes in viral quasispecies of the hepatitis C virus (HVC)

It is possible to interrogate any of the possible mutations in regions of the HCV lying between nucleotides 6967-7086 (ISDR, Interferon Sensitive Determining Region), 2319-2351 (taking as nucleotide 1 the first nucleotide of the viral genome) genotype 1-b and the regions (+/- 20 nt) around positions 195 and 330 where the two former correspond to the regions related to interferon response (IFN) and the last two to ribozymatic activity. The ISDR is directly involved in interferon response (N.Engl. J. Med.334:77-81), whereas the region 1978-2010 is associated with a possible interference mechanism in the action of IFN across the PKR (Science 285:107-109). In the ISDR resistance to IFN does not appear to be motivated by the selection of any specific mutation but the patients infected by a similar sequence to specific variant respond less well to IFN than sequences that have several mutations of this variant. With regards the ribozymes, there are no clinical data as yet although some are being studied at present in the first phase of clinical trials. Although, there is not yet any conclusive evidence concerning the effect of point mutations in these regions of HCV on the resistance to certain drugs, the detection of minority memory genomes could provide very interesting data about persistence of the virus in an infected organism. For example, as shown in Figure 1, there are significant differences between the genotype sequences 1-a and 1-b for the regions involved in the response to INF.

The procedure followed is:
1) Isolation, purification, and analysis of viral RNA is done according to Cabot et al., 2000 and Gerotto et al., 1999 and the kits that are used to extract RNA from HCV are from Roche (references: 2065193 and 2065231000).
2) Amplification by RT-PCR and/or nested PCR of the DNA fragments containing the regions to be studied, with the sets of oligonucleotides listed in Table II and using the nucleic acids extracted according to stage 1 as a template. The amplified fragments contain the genome regions of HCV type1-b (in relation to the first nucleotide of the viral genome) between positions 6967-7086 (ISDR), 2319-2351, 175-215 (ribozyme region 1) and 310-350 (ribozyme region 2).
3) Cloning of the fragments in a bacterial vector and determination of the sequence of the majority clone.
4) Amplification by PCR of the cloned fragments and labelling of the amplified fragments with 32P-γ-ATP and the polynucleotide kinase of T4.
5) Hybridisation of the labelled fragments with the fragments amplified directly from the nucleic acid of the quasispecies and with itself without labelling.
6) Fractionation of the hybrids formed by polyacrylamide gel electrophoresis in native conditions. The fragment labelled with itself is used as the control.
7) Identification of the existence of minority genomes by the number of mutations in relation to the sudden change in electrophoretic mobility.
8) Extraction of hybridised DNA from the polyacrylamide gel by elution. PCR amplification of the eluted fragments, sequenciation of the amplified fragments and comparison of the deduced nucleotide sequences.

### Example 6.-Method for the detection of memory genomes of the hepatitis B virus (HBV) that are carriers of mutations that confer resistance to drugs in treated patients.

Following the general procedure described in Example 2 with the appropriate modifications, a DNA microchip was designed that contains all the nucleotide positions that encode the fragment lying between amino acids 500 and 600 of the reverse transcriptase (RT) of HBV. This region contains the "YMDD motif" involved in HBV resistance to lamuvidin (3TC) (Allen et al., 1998). The results permit the presence of minority memory genomes with the change in the amino acid M552V (nucleotide substitution ATG→GTG) in patients that have been treated with 3TC to be detected.

## Claims

1. A method for designing an individual antiviral therapy for a subject based on the detection of minority memory genomes in a viral quasispecies responsible for viral resistance to a drug or drug combination, comprising:
a) detecting minority memory genomes in a viral quasispecies from a sample suspected to contain said minority memory genomes wherein said minority memory genomes are present in a proportion from 10⁻⁵ (0.001%) to less than 50% of said viral quasispecies, contain at least one mutation in comparison to the majority genome of said viral quasispecies, and provide information about the evolutionary history of said viral quasispecies in said subject,
b) detecting the existence of nucleotidic mutation associated to the resistance to antiviral drugs in said minority memory genomes, and
c) considering the data obtained in the previous steps for designing an individual antiviral therapy, so that it employs one or more antiviral drugs against which said viral quasispecies does not present resistance based on mutations on said minority memory genomes.

2. Method according to claim 1, wherein the detection of said minority memory genomes in a viral quasispecies, comprises:
a) extracting the nucleic acid from a sample suspected to contain said minority memory genomes in a viral quasispecies;
b) amplifying at least one nucleic acid fragment of said viral quasispecies; and
c) detecting and analysing the existence of minority memory genomes using techniques selected among the use of DNA microchips, the heteroduplex trace assay and molecular cloning.

3. Method according to claim 2, comprising:
a) extracting the nucleic acid from a sample suspected to contain said minority memory genomes in a viral quasispecies;
b) amplifying at least one nucleic acid fragment of said viral quasispecies;
c) labelling the amplified fragment or fragments with a marker compound;
d) constructing a DNA microchip comprising:
i) at least one oligonucleotide that serves as a positive control,
ii) at least one oligonucleotide that serves as a negative control,
iii) at least one memory oligonucleotide, and
iv) means that allow to draw up a calibration curve;
e) placing in contact said fragments amplified in stage b) and labelled in stage c) with the oligonucleotides present in the DNA microchip prepared in stage d) under conditions that permit hybridisation only when all the nucleotides of an oligonucleotide present in said DNA microchip pair with a nucleotide sequence present in said amplified and labelled fragments;
f) identifying the oligonucleotides present in said DNA microchip that have hybridised with said amplified and labelled fragments; ruling out negative hybridisations or background noise; and
g) selecting the oligonucleotides present in said DNA microchip that have hybridised with said amplified and labelled fragments and that by interpolation with the calibration curve show a proportion of said fragments in the quasispecies lower than 50% characteristic of minority memory genomes.

4. Method according to claim 3, wherein said minority memory genome is present in a proportion between 0.1% and 10% of the quasispecies.

5. Method according to claim 3, wherein said sample suspected to contain said minority memory genomes in a viral quasispecies is a sample selected from either a clinical sample or one derived from a viral culture.

6. Method according to claim 3, wherein said viral quasispecies belongs to the human immunodeficiency virus type-1 (HIV-1).

7. Method according to claim 3, wherein said viral quasispecies belongs to the human immunodeficiency virus type-2 (HIV-2).

8. Method according to claim 3, wherein said viral quasispecies belongs to hepatitis C virus (HCV).

9. Method according to claim 3, wherein said viral quasispecies belongs to the hepatitis B virus (HBV).

10. Method according to claim 3, wherein said viral quasispoeics belongs to the foot-and-mouth disease virus (FMDV).

11. Method according to claim 3, comprising carrying out a reverse transcription of viral RNA before the amplification stage b).

12. Method according to claim 3, wherein said amplification is done by enzymatic methods.

13. Method according to claim 12, wherein said enzymatic methods comprise the polymerase chain reaction (PCR), the ligase chain reaction (LCR) or the amplification based on transcription (TAS).

14. Method according to claim 3, wherein the fragment to be amplified in stage b) corresponds to a part or all of at least one gene essential for replication or persistence of the virus in the infected organism.

15. Method according to claim 14 wherein said essential gene is selected from the group comprised by: the protease fragment (PR) of the pol gene of HIV, the reverse transcriptase fragment (RT) of the *pol* gene of HIV, the integrase fragment of the *pol* gene of HIV, the *env* gene of HIV, the *gag* gene of HIV, the gene of the non-structural protein NS5A of HCV, the region between nucleotides 175-215 of HCV, the region between nucleotides 310-350 of HCV and the reverse transcriptase (RT) fragment of the pol gene of HBV.

16. Method according to claim 3, wherein said marker compound used to label the amplified fragments is selected from a radioactive compound, a fluorescent compound or a compound detectable by colorimetric reaction.

17. Method according to claim 3, wherein said DNA microchip is constituted by previously synthesized oligonucleotide points

18. Method according to claim 3, wherein said DNA microchip is composed of oligonucleotide points previously synthesized *in situ.*

19. Method according to claim 3, wherein said positive control comprises at least one oligonucleotide with at least one oligonucleotide sequence that is 100% complementary to a known sequence of the majority genome or of the wild type genome of the virus.

20. Method according to claim 3, wherein said negative control is selected by the group formed by: i) an oligonucleotide with a sequence that is complementary to a region of known sequence of the majority genome or wild type genome of the virus, except for at least one position; ii) an oligonucleotide with a sequence that is complementary to a known sequence region of the majority or wild type genome, except in the interrogant position; and iii) an oligonucleotide with a sequence that is complementary to a known sequence region of the majority or wild type genome except for the interrogant position and at least one flanking nucleotide of said interrogant position.

21. Method according to claim 3, wherein said memory oligonucleotide is selected from the group formed by:
- a nucleic acid with a length from 4 to 250 nt that is equal or complementary to a majority or average viral genome sequence except for the 1-6 central positions (MO1);
- a nucleic acid from 5-50 nt in length that is formed by stacking two oligonucleotides after hybridising with another complementary nucleic acid from the virus being one of the stagnant oligonucleotides made up of a mixture of four oligonucleotides that differ in the position immediately adjacent to the previous oligonucleotide and that carry a different fluorescent colouring covalently bound to the other end (MO2);
- a nucleic acid of between 5 and 250 nt comprised of two parts, one 5' complementary to the other oligonucleotide absent from the viral genome and a 3' part complementary to the viral genome, the last position being an interrogant position (MO3);
- a nucleic acid from 5 to 250 nt long complementary to the viral genome that has a fluorescent substance covalently bound to the 3' end (MO4);
- a nucleic acid from 5 to 250 nt long complementary to the viral genome of which the final position of the 3'end is anterior to an interrogated position of the viral genome (M05);
- a nucleic acid from 5 to 250 nt long complementary to a sequence of a majority genome of a viral quasispecies with insertions 1 to 10 nt with respect to the majority genome sequence (M06);
- a nucleic acid of between 5 and 250 nt complementary to a majority genome sequence of the viral quasispecies with deletions 1 to 10 nt with respect to this majority genome sequence (M07);
- a nucleic acid of 5 to 250 nt complementary to a mutant sequence previously described in the databases; and
- their mixtures.

22. Method according to claim 3, wherein said calibration curve is drawn up using a series of mixtures of oligonucleotides in variable and known proportions, one of these being 100% complementary to a region of known sequence of the majority genome or the wild type genome and being the other one an oligonucleotide that differs from the previous one in at least one position.

23. Method according to claim 3, wherein the calibration curve is formed by a series of mixtures of oligonucleotides in variable and known proportions, one of which being 100% complementary to a known sequence of the majority genome or the wild type genome and the other one being an oligonucleotide that differs from the previous one in the interrogant position.

24. Method according to claim 3, wherein the identification of the oligonucleotides present in the DNA microchip which have hybridised with the amplified and labelled fragments is done by scanning said microchip with a scanner equipped with a confocal microscope and at least two lasers which emit light of a different wavelength and computer equipment that can produce a computerised image of the hybridisation results.

25. Method according to claim 2, comprising:
a) extracting the nucleic acid from a sample suspected to contain said minority memory genomes in a viral quasispecies;
b) amplifying at least one nucleic acid fragment of said viral quasispecies;
c) cloning the DNA fragments amplified in stage b) into a suitable vector
d) determining the majority genome sequence of the viral quasispecies for the amplified fragment;
e) amplifying the DNA fragments cloned in stage c) and labelling the amplified fragments with a marker compound;
f) placing in contact, in a hybridisation reaction, the amplified and labelled fragments from stage e) with the fragments amplified directly from the nucleic acid of the viral quasispecies from stage b); and
g) resolving the different viral sequences and identifying the mutations indicative of the minority memory genomes present in the viral quasispecies.

26. Method according to claim 25, wherein the cloning of the DNA fragments amplified in stage b) is done in a plasmid with a large number of copies.

27. Method according to claim 25, wherein the marker compound used in stage e) to label the fragments of DNA amplified and cloned in stage c) is ³²P.

28. Method according to claim 25, wherein the resolution of the different viral sequences, stage g), is done by:
g.i) fractionating the hybrids formed in stage f) by polyacrylamide gel electrophoresis in non denaturising conditions;
g.ii) identifying the existence of minority memory genomes by the number of mutations in relation to a sudden change in electrophoretic mobility;
g.iii) extracting DNA hybridised and fractionated in polyacrylamide gel by elution;
g.iv) amplifying the fractions eluted in stage g.iii);
g.v) sequencing the fragments amplified in stage g.iv); and
g.vi) comparing the sequences deduced in stage g.v) and identifying the mutations indicative of the minority memory genomes present in the viral quasispecies.

29. Method according to claim 2, comprising:
a) extracting the nucleic acid from a sample suspected to contain said minority memory genomes in a viral quasispecies;
b) amplifying at least one nucleic acid fragment of said viral quasispecies;
c) determining the majority genome sequence of the viral quasispecies for the amplified fragment;
d) optionally, cloning the fragment of nucleic acid amplified in a vector;
e) sequencing the cloned fragment; and
f) comparing the sequences deduced in stage c) and e), and identifying the mutations indicative of the minority memory genomes present in the viral quasispecies.

## Patentansprüche

1. Verfahren zur Entwicklung einer individuellen antiviralen Therapie für einen Patienten basierend auf dem Nachweis von Minderheitsgedächtnisgenomen in einer viralen Quasi-Spezies, welche verantwortlich sind für die virale Resistenz gegenüber einem Arzneistoff oder einer Arzneistoffkombination, umfassend die Schritte:
(a) Nachweis der Minderheitsgedächtnisgenome in einer viralen Quasi-Spezies in einer Probe, welche im Verdacht steht, die Minderheitsgedächtnisgenome zu enthalten, wobei diese Minderheitsgedächtnisgenome in einem Anteil von 10⁻⁵(0,001 %) bis weniger als 50% der viralen Quasi-Spezies vorliegen, mindestens eine Mutation im Vergleich zum Mehrheitsgenom der viralen Quasi-Spezies enthalten und Informationen über die Entstehungsgeschichte der viralen Quasi-Spezies in dem Patienten liefern,
(b) Nachweis der Existenz von Nucleotidmutationen in dem Minderheitsgedächtnisgenomen, welche mit der Resistenz gegenüber antiviralen Arzneistoffen assoziiert sind, und
(c) Inbetrachtziehen der Daten, welche in den vorhergehenden Schritten erhalten wurden, zur Entwicklung einer individuellen antiviralen Therapie, wobei einer oder mehrere antivirale Arzneistoffe verwendet werden, gegen welche die virale Quasi-Spezies keine Resistenz basierend auf Mutationen in den Minderheitsgedächtnisgenomen aufweist.

2. Verfahren nach Anspruch 1, wobei der Nachweis der Minderheitsgedächtnisgenome in einer viralen Quasi-Spezies umfasst:
(a) Extraktion der Nucleinsäure aus einer Probe, welche im Verdacht steht, die Minderheitsgedächtnisgenome in einer viralen Quasi-Spezies zu enthalten;
(b) Amplifikation von mindestens einem Nucleinsäurefragment der viralen Quasi-Spezies; und
(c) Nachweis und Analyse der Existenz von Minderheitsgedächtnisgenomen unter Nutzung von Techniken ausgewählt aus der Verwendung von DNA-Mikrochips, Heteroduplex-Trace-Assay und molekularem Clonieren.

3. Verfahren nach Anspruch 2, umfassend:
(a) Extraktion der Nucleinsäure aus einer Probe, welche im Verdacht steht, die Minderheitsgedächtnisgenome in einer viralen Quasi-Spezies zu enthalten;
(b) Amplifikation von mindestens einem Nucleinsäurefragment von der viralen Quasi-Spezies;
(c) Markierung des amplifizierten Fragments oder der amplifizierten Fragmente mit einer Markerverbindung;
(d) Konstruktion eines DNA-Mikrochips, umfassend:
(i) mindestens ein Oligonucleotid, das als Positivkontrolle dient,
(ii) mindestens ein Oligonucleotid, das als Negativkontrolle dient;
(iii) mindestens ein Gedächtnisoligonucleotid, und
(iv) Hilfsmittel, die es erlauben, eine Eichkurve zu erstellen;
(e) Inkontaktbringen der amplifizierten Fragmente aus Schritt (b), welche gemäß Schritt (c) markiert worden sind, mit den Oligonucleotiden, welche auf dem DNA-Mikrochip vorhanden sind, der gemäß Schritt (d) hergestellt wurde, unter Bedingungen, die eine Hybridisierung nur dann erlauben, wenn alle Nucleotide eines Oligonucleotids auf dem DNA-Mikrochip mit einer Nucleotidsequenz paaren, welche in den amplifizierten und markierten Fragmenten vorhanden ist;
(f) Identifikation der Oligonucleotide, welche auf dem DNA-Mikrochip vorhanden sind und mit den amplifizierten und markierten Fragmenten hybridisiert haben; Ausschließen negativer Hybridisierungen und von Hintergrundrauschen; und
(g) Auswahl der Oligonucleotide, welche auf dem DNA-Mikrochip vorhanden sind und mit den amplifizierten und markierten Fragmenten hybridisiert haben und welche durch Interpolation mit der Eichkurve einen Anteil der Fragmente in der Quasi-Spezies zeigen, welcher zu weniger als 50% charakteristisch für Minderheitsgedächtnisgenome ist.

4. Verfahren nach Anspruch 3, wobei das Minderheitsgedächtnisgenom in einem Anteil zwischen 0,1% und 10% der Quasi-Spezies vorhanden ist.

5. Verfahren nach Anspruch 3, wobei die Probe, welche im Verdacht steht, die Minderheitsgedächtnisgenome in einer viralen Quasi-Spezies zu enthalten, entweder eine klinische Probe ist oder von einer viralen Kultur stammt.

6. Verfahren nach Anspruch 3, wobei die virale Quasi-Spezies zu dem menschlichen Immunschwächevirus vom Typ 1 (HIV-1) gehört.

7. Verfahren nach Anspruch 3, wobei die virale Quasi-Spezies zu dem menschlichen Immunschwächevirus vom Typ 2 (HIV-2) gehört.

8. Verfahren nach Anspruch 3, wobei die virale Quasi-Spezies zu dem Hepatitis C-Virus (HCV) gehört.

9. Verfahren nach Anspruch 3, wobei die virale Quasi-Spezies zu dem Hepatitis B-Virus (HBV) gehört.

10. Verfahren nach Anspruch 3, wobei die virale Quasi-Spezies zu dem Maul-und Klauenseuche-Virus (MKSV) gehört.

11. Verfahren nach Anspruch 3, umfassend den Schritt einer reversen Transkription der viralen RNA vor der Amplifikation gemäß Schritt (b).

12. Verfahren nach Anspruch 3, wobei die Amplifikation mit enzymatischen Verfahren durchgeführt wird.

13. Verfahren nach Anspruch 12, wobei die enzymatischen Verfahren die Polymerasekettenreaktion (PCR), die Ligasekettenreaktion (LCR) oder die Amplifikation basierend auf Transkription (TAS) umfassen.

14. Verfahren nach Anspruch 3, wobei das in Schritt (b) zu amplifizierende Fragment teilweise oder ganz mindestens einem Gen entspricht, welches für Replikation oder Verharren des Virus im infizierten Organismus essentiell ist.

15. Verfahren nach Anspruch 14, wobei das essentielle Gen ausgewählt ist aus der Gruppe umfassend: das Proteasefragment (PR) des *pol*-Gens von HIV, das Reverse Transkriptasefragment (RT) des *pol*-Gens von HIV, das Integrasefragment des *pol*-Gens von HIV, das *env*-Gen von HIV, das *gag*-Gen von HIV, das Gen des Nicht-Struktur-Proteins NS5A von HCV, den Bereich zwischen den Nucleotiden 175-215 von HCV, den Bereich zwischen den Nucleotiden 310-350 von HCV, und das Reverse Transkriptase (RT)-Fragment des *pol*-Gens von HBV.

16. Verfahren nach Anspruch 3, wobei die Markerverbindung, welche zur Markierung der amplifizierten Fragmente verwendet wird, ausgewählt ist aus einer radioaktiven Verbindung, einer fluoreszierenden Verbindung oder einer Verbindung, welche durch kolorimetrische Reaktion nachweisbar ist.

17. Verfahren nach Anspruch 3, wobei der DNA-Mikrochip aus zuvor synthetisierten Oligonucleotidpunkten besteht.

18. Verfahren nach Anspruch 3, wobei der DNA-Mikrochip aus zuvor *in situ* synthetisierten Oligonucleotidpunkten besteht.

19. Verfahren nach Anspruch 3, wobei die Positivkontrolle mindestens ein Oligonucleotid mit mindestens einer Oligonucleotidsequenz umfasst, welche zu 100% komplementär zu einer bekannten Sequenz des Mehrheitsgenoms oder des Wildtypgenoms des Virus ist.

20. Verfahren nach Anspruch 3, wobei die Negativkontrolle ausgewählt ist aus der Gruppe bestehend aus: (i) einem Oligonucleotid mit einer Sequenz, welche komplementär zu einem Bereich mit bekannter Sequenz des Mehrheitsgenoms oder Wildtypgenoms des Virus ist, ausgenommen an mindestens einer Position; (ii) einem Oligonucleotid mit einer Sequenz, welche komplementär zu einem Bereich mit bekannter Sequenz des Mehrheitsgenoms oder des Wildtypgenoms ist, ausgenommen an der unterschiedlichen Position; und (iii) einem Oligonucleotid mit einer Sequenz, welche komplementär zu einem Bereich mit bekannter Sequenz des Mehrheitsgenoms oder des Wildtypgenoms ist, ausgenommen die unterschiedliche Position und mindestens ein die unterschiedliche Position flankierendes Nucleotid.

21. Verfahren nach Anspruch 3, wobei das Gedächtnisoligonucleotid ausgewählt ist aus der Gruppe bestehend aus:
- einer Nucleinsäure mit einer Länge von 4 bis 250 Nucleotiden, welche gleich oder komplementär zu einer Mehrheits- oder einer durchschnittlichen viralen Genomsequenz ist, ausgenommen die 1-6 zentralen Positionen (M01);
- einer Nucleinsäure von 5 bis 50 Nuclootiden Länge, hergestellt durch Stapelung zweier Oligonucleotide nach Hybridisierung mit einer weiteren komplementären Nucleinsäure des Virus, welche eines der stagnierenden Oligonucleotide ist, das aus einer Mischung von 4 Oligonucleotiden besteht, die sich in der Position unterscheiden, welche unmittelbar benachbart zu dem vorhergehenden Oligonucleotid liegt, und welche einen anderen Fluoreszenzfarbstoff tragen, der kovalent an das andere Ende gebunden ist (M02);
- einer Nucleinsäure von zwischen 5 und 250 Nucleotiden Länge, umfassend zwei Teile, wovon einer 5' komplementär zu dem anderen Oligonucleotid ist, welches nicht Teil des viralen Genoms ist, und einer 3' komplementär zu dem viralen Genom ist, wobei die letzte Position eine unterschiedliche Position ist (M03);
- einer Nucleinsäure von 5 bis 250 Nucleotiden Länge, welche komplementär zu dem viralen Genom ist und einen Fluoreszenzfarbstoff kovalent an das 3'-Ende gebunden hat (M04);
- einer Nucleinsäure von 5 bis 250 Nucleotiden Länge, welche komplementär zu dem viralen Genom ist, von welcher die letzte Positon des 3'-Endes vor der fraglichen Position des viralen Genoms liegt (M05);
- einer Nucleinsäure von 5 bis 250 Nucleotiden Länge, welche komplementär zu einer Sequenz eines Mehrheitsgenoms einer viralen Quasi-Spezies ist, mit Insertionen von 1-10 Nucleotiden im Bezug auf die Mehrheitsgenomsequenz (M06);
- einer Nucleinsäure zwischen 5 und 250 Nucleotiden Länge, welche komplementär zu einer Sequenz des Mehrheitsgenoms der viralen Quasi-Spezies ist, mit Deletionen von 1 bis 10 Nucleotiden im Bezug auf diese Mehrheitsgenomsequenz (M07);
- einer Nucleinsäure von 5 bis 250 Nucleotiden Länge, welche komplementär zu einer mutierten Sequenz ist, die zuvor in den Datenbanken beschrieben wurde; und
- deren Gemische.

22. Verfahren nach Anspruch 3, wobei die Eichkurve **dadurch** erstellt wurde, dass eine Serie von Gemischen von Oligonucleotiden in variablen und bekannten Anteilen verwendet wurde, wobei eines dieser zu 100% komplementär zu einem Bereich mit bekannter Sequenz des Mehrheitsgenoms oder des Wildtypgenoms ist und das andere ein Oligonucleotid ist, welches sich von dem vorhergehenden in mindestens einer Position unterscheidet.

23. Verfahren nach Anspruch 3, wobei die Eichkurve **dadurch** erstellt wurde, dass eine Serie von Gemischen von Oligonucleotiden in variablen und bekannten Anteilen verwendet wurde, wobei eines dieser zu 100% komplementär zu einer bekannten Sequenz des Mehrheitsgenoms oder des Wildtypgenoms ist und das andere ein Oligonucleotid ist, welches sich von dem vorhergehenden an der unterschiedlichen Position unterscheidet.

24. Verfahren nach Anspruch 3, wobei die Identifikation der Oligonucleotide auf dem DNA-Mikrochip, welche mit den amplifizierten und markierten Fragmenten hybridisiert haben, **dadurch** erreicht wird, dass der Mikrochip in einem Scanner gescannt wird, der mit einem konfokalen Mikroskop und mit mindestens zwei Lasern, welche Licht verschiedener Wellenlänge emittieren, bestückt ist, sowie einer Computerausrüstung, die ein computergesteuertes Bild der Hybridisierungsresultate erstellen kann.

25. Verfahren nach Anspruch 2, umfassend:
(a) Extraktion der Nucleinsäure aus einer Probe, welche im Verdacht steht, die Minderheitsgedächtnisgenome in einer viralen Quasi-Spezies zu enthalten;
(b) Amplifikation von mindestens einem Nucleinsäurefragment der viralen Quasi-Spezies;
(c) Clonierung der DNA-Fragmente, welche in Schritt (b) amplifiziert wurden, in einen geeigneten Vektor;
(d) Ermittlung der Mehrheitsgenomsequenz der viralen Quasi-Spezies für das amplifizierte Fragment;
(e) Amplifikation der DNA-Fragmente, welche in Schritt (c) cloniert wurden, und Markierung der amplifizierten Fragmente mit einer Markerverbindung,
(f) Inkontaktbringen der amplifizierten und markierten Fragmente von Schritt (e) in einer Hybridisierungsreaktion mit den Fragmenten, welche direkt von der Nucleinsäure der viralen Quasi-Spezies aus Schritt (b) amplifiziert wurden; und
(g) Auftrennung der verschiedenen viralen Sequenzen und Identifikation der Mutationen, welche die Minderheitsgedächtnisgenome anzeigen, die in der viralen Quasi-Spezies vorhanden sind.

26. Verfahren nach Anspruch 25, wobei die Clonierung der DNA-Fragmente, die in Schritt (b) amplifiziert wurden, in einen Plasmidvektor mit einer großen Anzahl von Kopien erfolgt.

27. Verfahren nach Anspruch 25, wobei die Markerverbindung, welche in Schritt (e) zur Markierung der amplifizierten und clonierten DNA-Fragmente von Schritt (c) verwendet wird, ³²P ist.

28. Verfahren nach Anspruch 25, wobei die Auftrennung der verschiedenen viralen Sequenzen aus Schritt (g) erreicht wird durch:
(g.i) Fraktionierung der Hybride aus Schritt (f) durch Polyacrylamidgelelektrophorese unter nicht-denaturierenden Bedingungen;
(g.ii) Identifikation des Vorhandenseins von Minderheitsgedächtnisgenomen durch die Anzahl der Mutationen im Verhältnis zu einer plötzlichen Veränderung in der elekrophoretischen Mobilität;
(g.iii) Extraktion der hybridisierten DNA, die im Polyacrylamidgel fraktioniert wurde, durch Elution;
(g.iv) Amplifikation der Fraktionen, welche in Schritt (g.iii) eluiert wurden;
(g.v) Sequenzierung der Fragmente, welche in Schritt (g.iv) amplifiziert wurden; und
(g.vi) Vergleich der Sequenzen, die in Schritt (g.v) erhalten wurden, und Identifikation der Mutationen, welche die Minderheitsgedächtnisgenome anzeigen, die in der viralen Quasi-Spezies vorhanden sind.

29. Verfahren nach Anspruch 2, umfassend:
(a) Extraktion der Nucleinsäure aus einer Probe, welche im Verdacht steht, die Minderheitsgedächntisgenome in einer viralen Quasi-Spezies zu enthalten;
(b) Amplifikation von mindestens einem Nucleinsäurefragment der viralen Quasi-Spezies;
(c) Bestimmung der Sequenz des Mehrheitsgenoms der viralen Quasi-Spezies für das amplifizierte Fragment;
(d) fakultative Clonierung des amplifizierten Nucleinsäurefragments in einen Vektor;
(e) Sequenzierung des clonierten Fragments; und
(f) Vergleich der Sequenzen, die in den Schritten (c) und (e) erhalten wurden, und Identifikation der Mutationen, welche die Minderheitsgedächtnisgenome anzeigen, die in der viralen Quasi-Spezies sind.

## Revendications

1. Une méthode visant à concevoir une thérapie antivirale individuelle pour un sujet, sur la base de la détection de génomes-mémoire minoritaires dans une quasi-espèce virale responsable de la résistance virale à un médicament ou à une combinaison de médicaments, comprenant :
a) la détection des génomes-mémoire minoritaires dans une quasi-espèce virale à partir d'un échantillon suspecté de contenir lesdits génomes-mémoire minoritaires, où lesdits génomes-mémoire minoritaires sont présents dans une proportion allant de 10⁻⁵ (0,001 %) à moins de 50 % de ladite quasi-espèce virale, contenant au moins une mutation par rapport au génome majoritaire de ladite quasi-espèce virale, et fournisant des informations sur l'histoire évolutive de ladite quasi-espèce virale chez ledit suiet,
b) la détection de l'existence de mutation nucléotidique associée à la résistance aux médicaments antiviraux dans lesdits génomes-mémoire minoritaires, et
c) l'analyse des données collectées au cours des étapes précédentes pour concevoir une thérapie antivirale individuelle, de telle sorte qu'elle emploie un ou plusieurs médicaments antiviraux contre lesquels ladite quasi-espèce virale ne présente aucune résistance basée sur des mutations desdits génomes-mémoire minoritaires.

2. Méthode selon la revendication 1, dans laquelle la détection desdits génomes-mémoire minoritaires dans une quasi-espèce virale comprend :
a) l'extraction de l'acide nucléique d'un échantillon suspecté de contenir lesdits génomes-mémoire minoritaires dans une quasi-espèce virale ;
b) l'amplification d'au moins un fragment d'acide nucléique de ladite quasi-espèce virale ; et
c) la détection et l'analyse de l'existence de génomes-mémoire minoritaires à l'aide de techniques sélectionnées parmi l'utilisation de micropuces à ADN, le dosage de traces par hétéroduplex et le clonage moléculaire.

3. Méthode selon la revendication 2, comprenant :
a) l'extraction de l'acide nucléique d'un échantillon suspecté de contenir lesdits génomes-mémoire minoritaires dans une quasi-espèce virale ;
b) l'amplification d'au moins un fragment d'acide nucléique de ladite quasi-espèce virale ;
c) le marquage du ou des fragments amplifiés avec un composé de marquage ;
d) la construction d'une micropuce à ADN comprenant :
i) au moins un oligonucléotide qui sert de contrôle positif,
ii) au moins un oligonucléotide qui sert de contrôle négatif,
iii) au moins un oligonucléotide-mémoire, et
iv) un moyen permettant d'établir une courbe d'étalonnage ;
e) la mise en contact desdits fragments amplifiés en phase b) et marqués à l'étape c) avec les oligonucléotides présents dans la micropuce à ADN préparée à l'étape d) sous des conditions qui permettent l'hybridation uniquement lorsque tous les nucléotides d'un oligonucléotide présent dans ladite micropuce à ADN s'apparient avec une séquence nucléotidique présente dans lesdits fragments amplifiés et marqués.
f) l'identification des oligonucléotides présents dans ladite micropuce à ADN qui se sont hybridés avec lesdits fragments amplifiés et marqués : l'exclusion des hybridations négatives ou du bruit de fond ; et
g) la sélection des oligonucléotides présents dans ladite micropuce à ADN qui se sont hybridés avec lesdits fragments amplifiés et marqués et qui, par interpolation avec la courbe d'étalonnage, indiquent une proportion desdits fragments dans la quasi-espèce caractéristique à moins de 50 % des génomes-mémoire minoritaires.

4. Méthode selon la revendication 3, dans laquelle ledit génome-mémoire minoritaire est présent dans une proportion comprise entre 0.1 % et 10 % de la quasi-espèce.

5. Méthode selon la revendication 3, dans laquelle ledit échantillon suspecté de contenir lesdits génomes-mémoire minoritaires dans une quasi-espèce virale est un échantillon prélevé dans un échantillon clinique ou dérivé d'une culture virale.

6. Méthode selon la revendication 3, dans laquelle ladite quasi-espèce virale appartient au virus de l'immunodéficience humaine de type 1 (VIH-1).

7. Méthode selon la revendication 3, dans laquelle ladite quasi-espèce virale appartient au virus de l'immunodéficience humaine de type 2 (VIH-2).

8. Méthode selon la revendication 3, dans laquelle ladite quasi-espèce virale appartient au virus de l'hépatite C (VHC).

9. Méthode selon la revendication 3, dans laquelle ladite quasi-espèce virale appartient au virus de l'hépatite B (VHB).

10. Méthode selon la revendication 3, dans laquelle ladite quasi-espèce virale appartient au virus de la fièvre aphteuse (FA).

11. Méthode selon la revendication 3 , comprenant la réalisation d'une transcription inverse de l'ARN viral avant l'étape d'amplification b).

12. Méthode selon la revendication 3, dans laquelle ladite amplification est effectuée via des méthodes enzymatiques.

13. Méthode selon la revendication 12, dans laquelle lesdites méthodes enzymatiques comprennent l'amplification en chaîne par polymérase, la réaction en chaîne par ligase ou l'amplification basée sur la transcription.

14. Méthode selon la revendication 3, dans laquelle le fragment à amplifier à l'étape b) correspond à une partie ou à la totalité d'au moins un gène essentiel à la réplication ou à la persistance du virus dans l'organisme infecté.

15. Méthode selon la revendication 14, dans laquelle ledit gène essentiel est sélectionné dans le groupe comprenant : le fragment de la protéase du gène *pol* du VIH, le fragment de la transcriptase inverse du gène *pol* du VIH, le fragment de l'intégrase du gène *pol* du VIH, le gène env du VIH, le gène *gag* du VIH, le gène de la protéine non structurelle NS5A du VHC, la région entre les nucléotides 175-215 du VHC, la région entre les nucléotides 310-350 du VHC et le fragment de la transcriptase inverse du gène *pol* du VHB.

16. Méthode selon la revendication 3, dans laquelle ledit composé de marquage utilisé pour marquer les fragments amplifiés est sélectionné parmi un composé radioactif, un composé fluorescent ou un composé détectable par réaction colorimétrique.

17. Méthode selon la revendication 3, dans laquelle ladite micropuce à ADN est constituée des points oligonucléotidiques synthétisés au préalable.

18. Méthode selon la revendication 3, dans laquelle ladite micropuce à ADN est composée des points oligonucléotidiques synthétisés au préalable *in situ.*

19. Méthode selon la revendication 3, dans laquelle ledit contrôle positif comprend au moins un oligonucléotide avec au moins une séquence oligonucléotidique qui est 100 % complémentaire à une séquence connue du génome majoritaire ou du génome de type sauvage du virus.

20. Méthode selon la revendication 3, dans laquelle ledit contrôle négatif est sélectionné par le groupe formé par : i) un oligonucléotide avec une séquence qui est complémentaire à une région d'une séquence connue du génome majoritaire ou génome de type sauvage du virus, sauf pour au moins une position; ii) un oligonucléotide avec une séquence qui est complémentaire à une région de séquence connue du génome majoritaire ou de type sauvage, sauf dans la position discriminatoire ; et iii) un oligonucléotide avec une séquence qui est complémentaire à une région de séquence connue du génome majoritaire ou de type sauvage, sauf pour la position discriminatoire et au moins un nucléotide flanquant de ladite position discriminatoire.

21. Méthode selon la revendication 3, dans laquelle ledit oligonucléotide-mémoire est sélectionné dans le groupe formé par :
- un acide nucléique d'une longueur de 4 à 250 nt qui est égal ou complémentaire à une séquence d'un génome viral majoritaire ou moyen, sauf pour les positions centrales 1-6 (M01);
- un acide nucléique d'une longueur de 5-50 nt qui est formé en empilant deux oligonucléotides après les avoir hybridés avec un autre acide nucléique complémentaire du virus qui constitue l'un des oligonucléotides stagnants obtenu en mélangeant quatre oligonucléotides qui divergent dans la position directement adjacente à l'oligonucléotide précédent et qui portent un colorant fluorescent différent lié de façon covalente à l'autre extrémité (M02) ;
- un acide nucléique compris entre 5 et 250 nt et composé de deux parties, une partie 5' complémentaire à l'autre oligonucléotide absent du génome viral et une partie 3' complémentaire au génome viral, la dernière position étant une position discriminatoire (MO3) ;
- un acide nucléique d'une longueur de 5 à 250 nt complémentaire au génome viral qui contient une substance fluorescente liée de façon covalente à l'extrémité 3' (MO4) ;
- un acide nucléique d'une longueur de 5 à 250 nt complémentaire au génome viral dont la position finale de l'extrémité 3' est antérieure à une position discriminée du génome viral (MO5) ;
- un acide nucléique de 5 à 250 nt de long complémentaire à une séquence d'un génome majoritaire d'une quasi-espèce virale avec des insertions de 1 à 10 nt sur la séquence du génome majoritaire (MO6) ;
- un acide nucléique compris entre 5 et 250 nt complémentaire à la séquence d'un génome majoritaire de la quasi-espèce virale avec des délétions de 1 à 10 nt sur la séquence du génome majoritaire (MO7) ;
- un acide nucléique de 5 à 250 nt complémentaire à une séquence mutante décrite précédemment dans les bases de données ; et
- leurs mélanges.

22. Méthode selon la revendication 3, dans laquelle ladite courbe d'étalonnage est établie à l'aide d'une série de mélanges d'oligonucléotides dans des proportions variables et connues, dont l'une est 100 % complémentaire à une région d'une séquence connue du génome majoritaire ou du génome de type sauvage et l'autre est un oligonucléotide qui diffère du précédent dans au moins une position.

23. Méthode selon la revendication 3, dans laquelle la courbe d'étalonnage est formée par une série de mélanges d'oligonucléotides dans des proportions variables et connues, dont l'une est 100 % complémentaire à une région de séquence connue du génome majoritaire ou le génome de type sauvage et l'autre étant un oligonucléotide qui diffère du précédent dans la position discriminatoire.

24. Méthode selon la revendication 3, dans laquelle l'identification des oligonucléotides présents dans la micropuce à ADN qui se sont hybridés avec les fragments amplifiés et marqués est effectuée en lisant ladite micropuce à l'aide d'un scanner équipé d'un microscope confocal, au moins deux lasers qui émettent une lumière d'une longueur d'onde différente et un équipement informatique capable de produire une image numérisée des résultats de l'hybridation.

25. Méthode selon la revendication 2, comprenant :
a) l'extraction de l'acide nucléique d'un échantillon suspecté de contenir lesdits génomes-mémoire minoritaires dans une quasi-espèce virale ;
b) l'amplification d'au moins un fragment d'acide nucléique de ladite quasi-espèce virale ;
c) le clonage des fragments d'ADN amplifiés en phase b) dans un vecteur approprié ;
d) la détermination de la séquence du génome majoritaire de la quasi-espèce virale pour le fragment amplifié :
e) l'amplification des fragments d'ADN clonés à l'étape c) et le marquage des fragments amplifiés avec un composé de marquage ;
f) la mise en contact, dans une réaction d'hybridation, des fragments amplifiés et marqués à l'étape e) avec les fragments amplifiés directement à partir de l'acide nucléique de la quasi-espèce virale de l'étape b) ; et
g) la résolution des différentes séquences virales et l'identification des mutations indiquant les génomes-mémoire minoritaires présents dans la quasi-espèce virale.

26. Méthode selon la revendication 25, dans laquelle le clonage des fragments d'ADN amplifiés à l'étape b) est effectué dans un plasmide avec un grand nombre de copies.

27. Méthode selon la revendication 25, dans laquelle le composé de marquage utilisé à l'étape e) pour marquer les fragments d'ADN amplifiés et clonés à l'étape c) est ³²P.

28. Méthode selon la revendication 25, dans laquelle la résolution des différentes séquences virales, l'étape g), est effectuée via :
g.i) le fractionnement des hybrides formés à l'étape f) par électrophorèse en gel de polyacrylamide en conditions non dénaturantes ;
g.ii) l'identification de l'existence de génomes-mémoire minoritaires via le nombre de mutations apparues à la suite d'un changement soudain de la mobilité électrophorétique ;
g.iii) l'extraction de l'ADN hybridé et fractionné dans le gel de polyacrylamide par ébullition ;
g.iv.) l'amplification des fragments élués à l'étape g.iii) ;
g.v) le séquençage des fragments amplifiés à l'étape g.iv) ; et
g.vi) la comparaison des séquences obtenues à l'étape g.v) et l'identification des mutations indiquant les génomes-mémoire minoritaires dans la quasi-espèce virale.

29. Méthode selon la revendication 2, comprenant :
a) l'extraction de l'acide nucléique d'un échantillon suspecté de contenir lesdits génomes-mémoire minoritaires dans une quasi-espèce virale ;
b) l'amplification d'au moins un fragment d'acide nucléique de ladite quasi-espèce virale ;
c) la détermination de la séquence du génome majoritaire de la quasi-espèce virale pour le fragment amplifié ;
d) éventuellement, le clonage du fragment d'acide nucléique amplifié dans un vecteur;
e) le séquençage du fragment cloné; et
f) la comparaison des séquences obtenues aux étapes c) et e) et l'identification des mutations indiquant les génomes-mémoire minoritaires présents dans la quasi-espèce virale.
